# NEW EUROPEAN PATENT SPECIFICATION

(11) **EP 1 908 770 B2**
(45) Date of publication and mention of the opposition decision: **15.07.2015**
(45) Mention of the grant of the patent: 30.11.2011
(21) Application number: 07124028.7
(22) Date of filing: 17.12.2001
(51) Int. Cl.: C07K 7/08, A61K 38/10, A61P 31/04

(54) **Methods for preparing purified lipopeptides**
Verfahren zur Herstellung gereinigter Lipopeptide
Procédés pour la préparation de lipopeptides purifiés

(30) Priority: 18.12.2000 US 256268 P; 09.03.2001 US 274741 P; 13.12.2001 US 340525 P; 13.12.2001 US 341315 P
(43) Date of publication of application: 09.04.2008
(62) Divisional of application: 01994272.1
(73) Proprietor: Cubist Pharmaceuticals, Inc., Lexington, MA 02421 (US)
(72) Inventor: Keith, Dennis, Montclair, MA 07042 (US); Lai, Jan-ji, Westborough, MA 01581 (US); Govardhan, Chandrika, Lexington, MA 02420 (US); Khalaf, Nazer, Worcester, MA 01602 (US)
(74) Representative: Cockerton, Bruce Roger

(56) References cited:
- EP-A- 0 337 731
- EP-A- 0 511 866
- EP-A1- 2 236 513
- EP-A2- 0 294 990
- WO-A1-99/43700
- WO-A1-02/059145
- WO-A2-01/53330
- WO-A2-02/096936
- DE-B- 1 021 538
- US- - 60/2 56, 268
- US- - 60/2 74, 741
- US-A- 4 439 425
- US-A- 5 336 756
- US-A- 5 912 226
- JANCARIK J ET AL: "SPARSE MATRIX SAMPLING: A SCREENING METHOD FOR CRYSTALLIZATION OF PROTEINS" JOURNAL OF APPLIED CRYSTALLOGRAPHY, COPENHAGEN, DK, vol. 24, 1991, pages 409-411, XP001053042 ISSN: 0021-8898
- E. NAOMI, CHAYEN: 'Recent advances in methodology for the crystallization of bioligical macromolecules' JOURNAL OF CRYSTAL GROWTH vol. 198/199, 1999, pages 649 - 655
- DUCRUIX A. ET AL: 'Crystallization of Nucleic Acids and Proteins A Practical Approach', vol. 2, 1999, OXFORD UNIVERSITY PRESS INC., NEW YORK pages 92 - 95
- JANCARIK J. ET AL: 'Sparse matrix sampling: a screening method for crystallization of proteins' J. APPL. CRYST. vol. 24, 1991, pages 409 - 411
- CARTER C.W. ET AL: 'Protein Crystallization Using Incomplete Factorial Experiments' THE JOURNAL OF BIOLOGICAL CHEMISTRY vol. 254, no. 23, 10 December 1979, pages 12219 - 12223
- Response filed during examination - 4.3.2011
- Technical Sheet Emerald Bio
- EVDOKIMOV A.G. ET AL: 'Overproduction, purification, crystallization and preliminary X-ray diffraction analysis of YopM, an essential virulence factor extruded by the plague bacteriumYersinia pestis' ACTA CRYST. 27 September 2000, pages 1676 - 1679
- Response filed during examination - 16.2.2011
- Prof. Dr.-Ing. Ulrich Vollrath Declaration
- US 6624143 B1 (D19 ENGLISH LANGUAGE TRANSLATION)
- Henry Gu Declaration
- Dr. Thomas Kelleher's contract of employment (redacted)
- Dr. Jan-Ji Lai's contract of employment (redacted)
- Structure of amphomycin
- Opponent's experiments (Example 12)
- BODANSZKY M. ET AL: 'Structure of the Peptide Antibiotic Amphomycin' JOURNAL OF THE AMERICAN CHEMICAL SOCIETY vol. 95, no. 7, 04 April 1973, pages 2352 - 2357
- Declaration from Dr. Lai

## Description

### TECHNICAL FIELD OF THE INVENTION

The present invention relates to methods of purifying daptomycin.

### BACKGROUND OF THE INVENTION

The rapid increase in the incidence of gram-positive infections-including those caused by antibiotic-resistant bacteria has sparked renewed interest in the development of novel classes of antibiotics. One such class is the lipopeptide antibiotics, which includes daptomycin. Daptomycin has potent bactericidal activity *in vitro* against clinically relevant gram-positive bacteria that cause serious and life-threatening diseases. These bacteria include, but are not limited to, resistant pathogens, such as vancomycin-resistant enterococci (VRE), methicillin*-*resistant *Staphylococcus aureus* (MRSA), glycopeptide intermediary susceptible *Staphylocuccus aureus* (GISA), coagulase-negative staphylococci (CNS), and penicillin-resistant *Streptococcus pneumoniae* (PRSP), for which there are very few therapeutic alternatives. See, *e.g.,* Tally at al.,1999, Exp, Opin. Invest, Drugs 8:1223-1238. Daptomycin's inhibitory effect is a rapid, concentration-dependent bactericidal effect *in vitro* and *in vivo,* and a relatively prolonged concentration-dependent post-antibiotic effect *in vivo.*

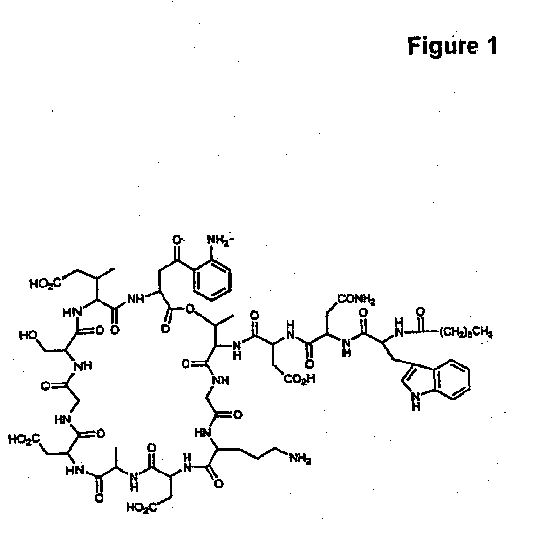

Daptomycin is described by Baltz in Biotechnology of Antibiotics, 2nd Ed., ed. W.R. Strohl (New Yore Marcel Dekker, Inc.), 1997, pp. 415-435. Daptomycin, also known as LY 146032, is a cyclic lipopeptide antibiotic that can be derived from the fermentation of *Streptomyces roseosporus.* Daptomycin is a member of the factor A-21978C₀ type antibiotics of *S. roseosporus* and is comprised of a decanoyl side chain linked to the N-terminal tryptophan of a cyclic 13-amino acid peptide (Fig. 1). Daptomycin has an excellent profile of activity because it is highly effective against most gram-positive bacteria; it is highly bactericidal and fast-acting; it has a low resistance rate and is elective against antibiotic-resistant organisms. The compound is currently being developed in a variety of formulations to treat serious infections caused by bacteria, including, but not limited to, methicillin-resistant *Staphylococcus aureus* (MRSA) and vancomycin-resistant enterococci (VRE).

A number of United States Patents describe A-21978C0 antibiotics and daptomycin-related lipopeptides including daptomycin (LY 146032). These patents also describe methods of producing and isolating the A-21978C₀ antibiotics and daptomycin-related lipopeptides.

United States Patents RE32,333, RE32,455, 4,800,157, 4,874,843, and 4,885,243 describe methods of synthesizing and isolating daptomycin from fermentation cultures of *Streptomyces roseosporus.* United States Patents RE32;310, RE32,311, 4,537,717, 4,482,487 and 4,524,135 describe A-21978C0 antibiotics and methods of deacylating the A-21978C₀ antibiotic and reacylating the peptide nucleus and antibiotic derivatives made by this process. United States Patent 5,912,226 (hereafter the '226 patent) describes the identification and isolation of two impurities produced during the manufacture of daptomycin, anhydro-daptomycin and the β-isomer form of daptomycin. None of these United States patents discloses a method for precipitating or crystallizing a lipopeptide in a manner to increase purity of the lipopeptide.

United States Patent 4,439,425 (hereafter the '425 patent) discloses a crystalline lipopeptide and a method of crystallizing the lipopeptide. The lipopeptide disclosed in the '425 patent is structurally dissimilar from daptomycin and daptomycin-related lipopeptides. United States Patent 5,336,756 (hereafter the '756 patent) also discloses a crystalline cyclic lipopeptide comprising a hexapeptide. The crystalline cyclic lipopeptide disclosed in ths '756 patent is also structurally dissimilar from daptomycin and daptomycia-related lipopeptides. The '756 patent discloses that the lipopeptide, am echinocandin-type compound, can be obtained when aqueous n-propanol is employed as the crystallizing solvent See, e.g., cols. 1-2 of the '756 patent Neither the '425 patent nor the '756 patent disclose methods of crystallizing or precipitating daptomycin or a daptomycin-related lipopeptide, nor do they disclose methods of crystallizing or precipitating lipopeptides produced by *Streptomyces.*

Jancarik + Kim, J. Appl. Cryst. (1991) 24 : 409-411, describe a generalised set of screening conditions which may be used for protein crystallisation.

It would be advantageous to develop a method of crystallizing or precipitating daptomycin and daptomycin-related lipopeptides to provide an improved purification method for these lipopeptides. In addition, a crystalline or highly purified precipitated form of daptemycin or other daptomycin-related lipopeptide would be user in formulating pharmaceutical compositions for treating bacterial infections. Further, a crystalline or highly purified precipitated form of daptomycin or daptomycin-related lipopeptide would be useful in an method to make a sterile product; particularly bulk sterile product Thus, there is a need for methods to produce crystalline or precipitated daptomycin and daptomycin-related lipopeptides and the crystalline or precipitated forms of the lipopeptides produced thereby. However, there has been no simple and robust method that has been effective in crystallizing or precipitating daptomycin or a daptomycin-related lipopeptide that results in a lipopeptide that is more pure alter crystallization or precipitation than before.

### SUMMARY OF THE INVENTION

The present invention provides a method of purifying daptomycin, in accordance with claim 1. Other features of the invention are described in the dependent claims.

### BRIEF DESCRIPTION OF THE DRAWINGS

Fig. 1 shows the structure of daptomycin.
Fig. 2 shows a photomicrograph of urchin-like crystal or crystal-like particle of daptomycin produced by the method described in Example 12.
Fig. 3 shows a photomicrograph of needle-like crystals of daptomycin.
Fig. 4 shows a photomicrograph of rod-like crystals of daptomycin.
Fig. 5 shows photomicrographs of daptomycin samples at 100X magnification. Photomicrographs of amorphous daptomycin are shown using plane transmitted light (A) and using crossed polarized light (B). Photomicrographs of daptomycin crystals are shown using plane transmitted light (C and B) and using crossed polarized light (D and F). The daptomycin crystals were produced by the protocol disclosed in Example 7.
Fig. 6 shows an x-ray powder diffraction pattern for amorphous daptomycin.
Fig. 7 shows an x-ray powder diffraction pattern for a daptomycin crystal produced by the protocol described in Example 7.
Fig 8 shows an x-ray powder diffraction pattern for a second sample of a daptomycin crystal produced by the protocol described in Example 7.
Fig. 9 shows birefringence of a crystal-like particle of daptomycin when exposed to polarized light The crystal-like particle was produced by the method described in Example 12.
Fig 10 shows a flow chart of an exemplary method for ciysw&ation.

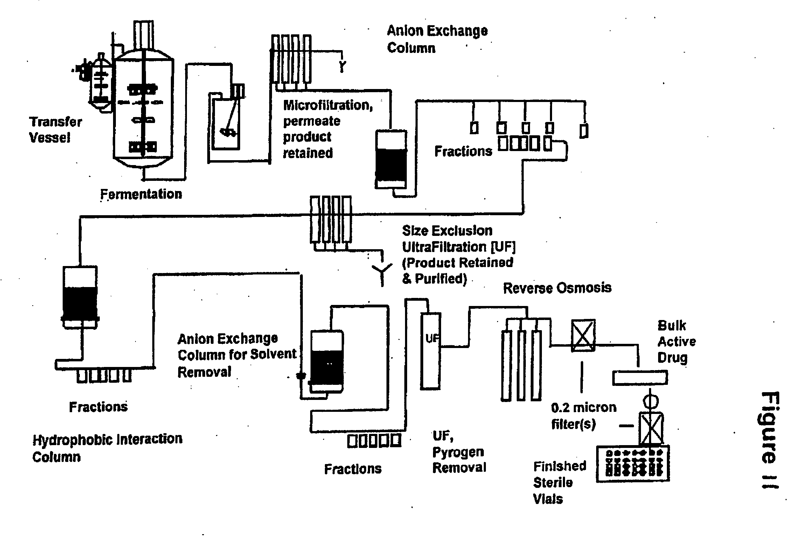

Fig. 11 shows a flow chart of an exemplary manufacturing method that does not use crystallization or precipitation. The manufacturing method uses bacterial fermentation to produce a fomentation culture containing daptomycin, and then purification of daptomycin using microfiltration, union exchange chromatography, size exclusion ultrafiltration, hydrophobic interaction chromatography, anion exchange chromatography for solvent removal, ultrafiltration for pyrogen removal, reverse osmosis and filling vials with daptomycin. See, e.g., International PCT Publication WO 01144274, published June 21, 2001 for a detailed description of this type of method.
Fig 12 shows a flow chart of an exemplary manufacturing method of a lipopeptide compound comprising the steps of fermentation, microfiltration, anion exchange chromatography, size exclusion ultrafiltration, crystallization or precipitation, crystal or precipitate drying, and dry filling of vials with the compound. See, e.g., Example 13.
Fig.13 shows a flow chart of an exemplary manufacturing method af a lipopeptide compound comprising the steps of fermentation, microfiltration, anion exchange chromatography, crystallization or precipitation, crystal or precipitate drying, and dry filling of vials with the compound See, e.g., Example 14.
Fig. 14 shows a flow chart ofan exemplary manufacturing method of a lipopeptide compounds comprising the steps of fermentation, microfiltration, size exclusion ultrafiltration, crystallization or precipitation, crystal or precipitate drying, and dry filling of vials with the compound. See, e.g., Example 15.
Fig. 15 shows a flow chart of an exemplary manufacturing method of a lipopeptide compound comprising the steps of fermentation, microfiltration, crystallization or precipitation, crystal or precipitate drying, and dry filling of vials with the compound. See, e.g., Example 16.

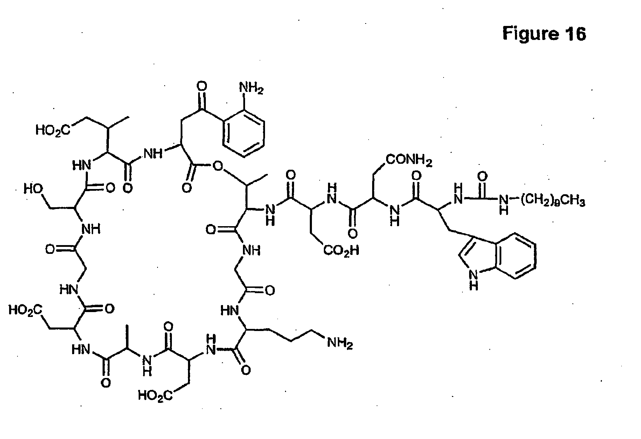

Fig. 16 depicts the structure of CB-131547, acyclic lipopeptide analog of daptomycin

### DETAILED DESCRIPTION OF THE INVENTION

One object of the present disclosure is to provide methods for crystallizing or precipitating lipopeptides. In one embodiment, the methods are used to crystallize or precipitate daptomycin or a daptomycin-related lipopeptide. In another embodiment, the methods increase the purity of the lipopeptide compared to the purity of the lipopeptide prior to crystallization or precipitation. The methods comprise the steps of providing an amorphous preparation ofa lipopeptide and crystallizing or precipitating the lipopeptide under conditions in which the crystalline or precipitated, crystal-like lipopeptide is more pure than the amorphous preparation of the lipopeptide. In one embodiment, the amorphous preparation is no greater than 92% pure and the crystalline or crystal-like lipopeptide purified therefrom is at least 95% pure, and may be at least 96% 97% or 98% or more pure. In another embodiment, the amorphous preparation is no greater than 80% pure and the crystalline or crystal-like lipopeptide purified therefrom is at least 95% pure, and may be at least 96%, 97% or 98% or more pure. In another embodiment, the amorphous preparation is no greater than 60% pure and the crystalline or crystal-like lipopeptide purified therefrom is at least 95% pure, and may be at least 96%, 97% or 98% ore more pure. In yet another embodiment, the amorphous preparation is no greater than 40% pure and the crystalline or crystal-like lipopeptide purified therefrom is at least 95% pure, and may be at least 96%, 97% or 98% or more pure. In another embodiment, the amorphous preparation is no greater than 20% pure and the crystalline or mystal-like lipopeptide purified therefrom is at least 95% pure, and may be at least 96%, 97% or 98% or mora pure. In a further preferred embodiment, the amorphous preparation is no greater than 10% pure and the crystalline or crystal-like lipopeptide purified therefrom is at least 95% pure, and may be at last 96%, 97% or 98% or more pure.

Another object of the present disclosure is to provide processes for making and purifying a lipopeptide comprising, *inter alia.* crystallizing or precipitating the lipopeptides. In one embodiment the crystallizing or precipitating steps are used to purify the lipopeptides. In a preferred embodiment, the crystallization or precipitation is performed by batch crystallized or precipitation. In another embodiment, the process is a large-scale process for commercial production of a lipopeptide, preferably daptomycin or a daptomycin-related lipopeptide. In one embodiment, the lipopeptide is produced by fermentation. The fermentation product is then purified by a variety of purification techniques including crystallization or precipitation. In one embodiment, the crystallization or precipitation step may be used in combination with other purification techniques including microfiltration, size exclusion ultrafiltration and/or anion exchange chromatography. In one embodiment; the crystallization or precipitation step is used to replace one or more purification techniques that is used in a purification process that does not use crystallization or precipitation. In another embodiment; the crystallization or precipitation step is used to increase purification compared to the other steps without the crystallization or precipitation step. In a preferred embodiment, the method comprises a step of collecting the crystalline or cxystal-like lipopeptide after crystallization or precipitation.

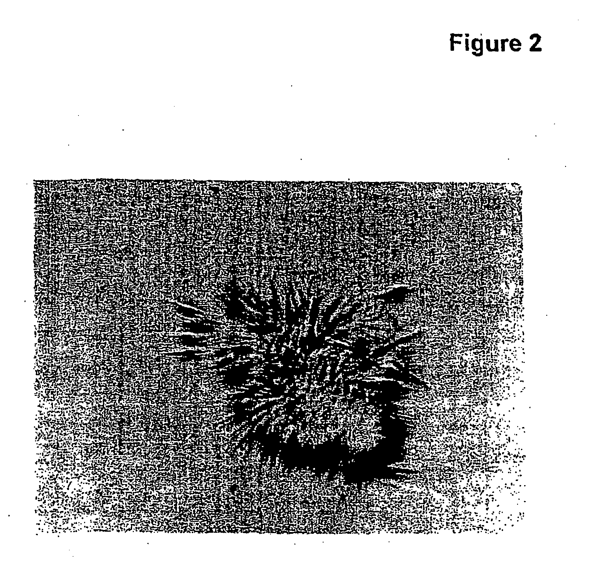

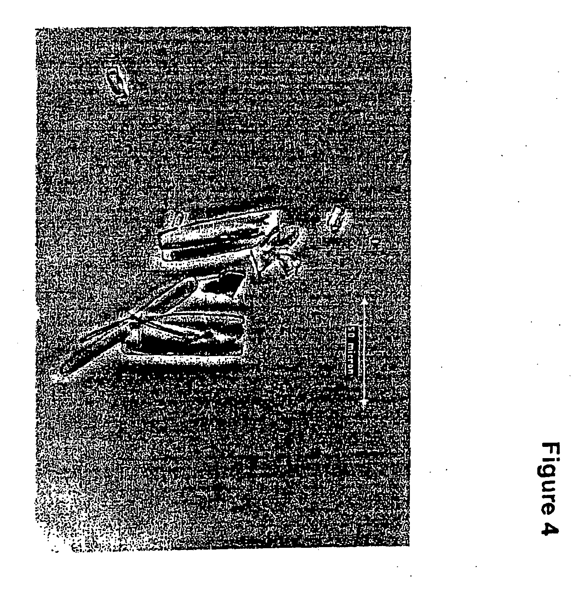

Another object of the present disclosure is to provide highly purified, e.g. steals, crystalline or crystal-like forms of lipopeptides. In one embodiment, the lipopeptides are daptomycin or a daptomycin-related lipopeptide. The crystalline or crystal-like form of the lipopeptide may have any crystalline or crystal-like shape including urchin-like (cluster of needles joined to gether to visually resemble a sea urchin)(see Fig. 2), needle-like (see Fig, 3), rod-like (see Fig 4), plate-like or flake-like. In one embodiment, the crystalline or crystal-like lipopeptide has a purity of at least 80%, and may be at least 85%, 90% pure. In another embodiment, the crystalline or crystal-like form of the lipopeptide has a purity of at least 95% and may be at least 96%, 97%. 98% pure or more.

A further object of the present disclosure is to provide a pharmaceutical composition comprising a crystalline or crystal-like form of a lipopeptide. In one embodiment; the lipopeptide is daptomycin or a daptomycin-related lipopeptide. In one embodiment, the pharmaceutical comp. is enterically coated for oral administration or is formulated in the form of micronized particles or microspheres. In other embodiments, the provides methods for administering the pharmaceutical compositions to subjects in need thereof.

### Definitions

Unless otherwise defined, all technical and scientific terms used herein have the meaning as commonly understood by one of ordinary skill in the art to which this invention belongs. The practice of the present invention employs, unless otherwise indicated, conventional techniques of chemistry, biochemistry, biophysics and microbiology and basic terminology used therein.

The term "lipopeptide" refers to a molecule that comprises a lipid-like moiety covalently linked to a peptide moiety, as well as salts, esters, amides and ethers thereof The term "lipopeptide" also encompasses protected forms of lipopeptides in which one or more amino, carboxylate or hydroxyl groups are protected. See, e.g., "Protective Groups in Organic Synthesis" by Theodora W. Greenery John Wiley and Sons, New York, 1981 for examples of protecting groups. In one embodiment, the lipopeptide is an antibiotic. In another embodiment, the lipopeptide is LY 303366, echinocandins, pneumocandins, aculeacins, viscosin, surfactin, plipastatin B1, amphomycin or the lipopeptide derivative disclosed in United States Patent 5,629,288. These lipopeptides are known in the art. See, e.g, United States Patent 5,202,309 and International PCT Application WO 00/08197. In another embodiment, the lipopeptide is a daptomycim-related molecule. In another embodiment, tha lipopeptide is daptomycin.

A "daptomycin-related molecule" includes, *inter alia,* daptomycin. A54145 or other lipopeptide that is structurally related to daptomycin, such as a daptomycin-related lipopeptide, including all stereoisomers that may be made at any chiral centers present in these molecular.

A "daptomycin-related lipopeptide" includes, without limitation, a lipopeptide disclosed in United States Patent 4,537,717, 4,482,487, RB32.311, RE32,310, and 5,912,226, currently in reissue as United States Application No. 09/547,357. Daptomycin-related lipopeptides also include those disclosed in International PCT Publication WO 01/44272, published June 21, 2001; International PCT Publication WO 01/44274, published June 21, 2001; and International PCT Publication WO 01144271, published June 21, 2001. The daptomycin-related lipopeptides disclosed in the above-identified applications relate to synthetic and semisynthetic lipopeptides in which the ornithine and/or kynurine residues, and/or the fatty acid side chain of daptomycin, are modified. Daptomycin-ralaed lipopeptides further include an A-21978Co antibiotic in which the n-decanoyl fatty acid side chain of daptomycin is replaced by a n-octanoyl, n-nonanoyl, n-undecanoyl, n-dodecanoyl, n-tridecenoyl or n-tetradecanoyl fatty acid side chain.

The term "daptomycin" refers to the n-decanoyl derivative of the factor A-21978C₀-type antibiotic that contains an α-aspartyl group. "Daptomycin" is synonymous with LY 146032.

The term "anhydro-daptomycin" refers to a daptomycin-related lipopeptide in which an α-aspartyl group of daptomycin is cyclized to a succinimido group. See, e.g., the '226 patent for the structure of anhydro-daptomycin.

The term "β-isomer" or "β"-isomer of daptomycin" refers to a dagiomycin-related lipopeptide that contains a β-aspartyl group instead of an α-aspartyl group. See, e.g, the '226 patent for the structure of β-isomer of daptomycin.

The term "isolated" refers to a compound or product that is at least 5%, 10%, 20%, 30%, 40%, 50%, 60%, 70%, 80% or 90% of the compound present in a mixture. It will be understood that the term "isolated" also refers to a compound that is at least 5-10%, 10-20%, 20-30%, 30-40%, 40-50%, 50-60%, 60-70%, 70-80% or 80-90% of the compound present in the mixture group. The percentage of compound in a mixture may be measured by any means known in the art, as described below for measuring purity of a compound.

"Substantially pure" refers to a sample having at least 95% of a desired compound. Preferably, daptomycin is "substantially pure" when at least 95% to at least 97% of a sample is daptomycin. Similarly, a daptomycin-related lipopeptide, is "substantially pure" when at least 95% to at least 97% of a sample is a daptomycin-related lipopeptide.

Daptomycin or a daptomycin-related lipopeptide is "essentially pure" when at least 98% to at least 99% of a sample is daptomycin or a daptomycin-related lipopeptide, respectively.

Daptomycin or a daptomycin-related lipopeptide is "substantially free" of another compound when the other compound is present in an amount that is no more than 1% of the amount of the daptomycin or the daptomycin-related lipopeptide preparation, respectively.

Daptomycin or a daptomycin-related lipopeptide is "essentially free" of another compound when the other compound is present in an amount that is no more than 0.5% of the amount of the daptomycin or the daptomycin-related lipopeptide preparation, respectively.

Daptomycin or a daptomycin-related lipopeptide is "free" of another compound when the other compound is present in an amount that is no more than 0.1% of the amount of the daptomycin or the daptomycin-related lipopeptide preparation, respectively. Alternatively, daptomycin or a daptomycin-related lipopeptide is "free" of another compound when the compound cannot be detected by HPLC under conditions of maximum sensitivity in which a limit of detection is approximately 0.05% or less of the amount of the daptomycin or the daptomycin-related lipopeptide preparation, respectively.

"Purified" daptomycin refers to substantially pure daptomycin, essentially pure daptomycin, or a salt thereof, or to daptomycin or a salt thereof which is substantially free, essentially free, or free of another compound. Similarly, a "purified" daptomycin-related lipopeptide refers to a substantially pure daptomycin-related lipopeptide, an essentially pure daptomycin-related lipopeptide, or a salt thereof, or to a daptomycin-related lipopeptide or a salt thereof which is substantially free, essentially free, or free of another compound.

"Crude" daptomycin refers to daptomycin or a salt thereof that is less than 90% pure. Similarly, "crude" daptomycin-related lipopeptide refers to a daptomycln-related lipopeptide or a salt thereof that is less than 90% pure.

"Semi-purified" daptomycin refers to daptomycin or a salt thereof that is at least 90% pure and less than 95% pure. Similarly, "semi-purified" daptomycin-related lipopeptide refers to a daptomycin-related lipopeptide or a salt thereof that is at least 90% pure and less than 95% pure.

The purity of daptomycin, daptomycin-related lipopeptide or of another lipopeptide refers to the lipopeptide prior to its formulation in a pharmaceutical composition. The purity of the lipopeptide is referred to by "percent purity." The measure of purity is not a measure of degree of crystallinity of the crystalline preparation. The purity may be measured by any means including nuclear magnetic resonance (NMR), gas chromatography/mass spectroscopy (GC/MS), liquid chromatography/mass spectroscopy (LC/MS) or microbiological assays. One preferred means for measuring the purity of daptomycin is by analytical high pressure liquid chromatography (HPLC). Two methods of analytical HPLC are described in International PCT Publication WO 01/53330, published July 26, 2001.

A "lipopeptide crystal" refers to one or more crystals of a lipopeptide or of a lipopeptide salt. The determination of a lipopeptide as a crystal can be determined by any means including, *inter alia*, optical microscopy, electron microscopy, x-ray powder diffraction, solid state nuclear magnetic resonance (NMR) or polarizing microscopy. Microscopy can be used to determine the crystal length, diameter, width, size and shape, as well as whether the crystal exists as a single particle or is polycrystalline.

A lipopeptide or lipopeptide particle is "crystal-like" if it is determined to have crystalline characteristics when determined by one means, e.g., visually or by optical or polarizing microscopy, but does not have crystalline characteristics when determined by another means, e.g., x-ray powder diffraction. A lipopeptide that is "crystal-like" may be crystalline under certain conditions but may become non-crystalline when subjected to other conditions.

A "crystalline lipopeptide" or a "crystalline form of a lipopeptide" refers to a preparation of a lipopeptide or salt thereof that comprises lipopeptide crystals. In one embodiment, a crystalline lipopeptide may comprise some amount of amorphous lipopeptide. In one embodiment, the crystalline lipopeptide comprises more than 50% by weight of lipopeptide crystals. In another embodiment, the crystalline lipopeptide comprises more than 60%, 70%, 80%, 90% or 95% of lipopeptide crystals. The crystalline lipopeptide may comprise 50-60%, 60-70%, 70-80%, 80-90% or 90-95% of lipopeptide crystals. In another embodiment, the crystalline lipopeptide comprises more than 95% of lipopeptide crystals, e.g., at least 96%, 97%, 98% or 99% lipopeptide crystals or 100% lipopeptide crystals. The crystalline lipopeptide may also comprise anywhere from 95-100% lipopeptide crystals. The percent by weight of lipopeptide crystals refers to the lipopeptide preparation prior to its formulation in a pharmaceutical composition.

An "amorphous" form of a lipopeptide refers to a lipopeptide preparation that comprises few or no lipopeptide crystals or crystal-like lipopeptides (or crystal-like particles) as defined herein. In one embodiment, an amorphous lipopeptide comprises less than 20% by weight of lipopeptide crystals or crystal-like lipopeptides. In another embodiment, an amorphous lipopeptide comprises less than 10% by weight of lipopeptide crystals or crystal-like lipopeptides. In another embodiment, an amorphous lipopeptide comprises less than 5% by weight of lipopeptide crystals or crystal-like lipopeptides. In a still further preferred embodiment, an amorphous lipopeptide comprises less than 1 % by weight of lipopeptide crystals or crystal-like lipopeptides.

"Batch crystallization" refers to a method in which the lipopeptide of interest is mixed with the crystallization reagents in solution and the lipopeptide is allowed to crystallize in solution. "Batch precipitation" refers to a method in which the lipopeptide is mixed with precipitation reagents in solution and the lipopeptide is allowed to precipitate in solution. In one embodiment, the crystalline or precipitated preparation is collected from the solution. In another embodiment, the crystalline or precipitated preparation is collected by filtration or centrifugation.

"Organic precipitant" refers to a polyethylene glycol (PEG) or polyethylene glycol monomethyl ether (PEG MME) or compounds that are chemically similar.

"Salts" refer to ionic compounds. These ionic compounds may act as precipitants.

"Low molecular weight alcohols" are organic compounds containing at least one alcohol functional group, and eight carbon atoms or less. For example, low molecular weight alcohols include, without limitation, methanol, isopropanol, and *tert*-butanol.

"Polyhydric alcohols" refer to compounds that contain more than one alcohol group, and less than eight carbon atoms. Polyhydric alcohols, for example, include, without limitation, 1,6 hexanediol, ethylene glycol, propylene glycol, glycerol, 1,2-propanediol, 2-methyl-2,4-pentanediol and 1,4 butanediol.

"Container" refers to a receptacle for holding goods. For example, a container may include, without limitation, an ampule, vial, tube, bottle, or cylinder.

### Methods of Producing Purified Lipopeptides

The claimed invention is set out in claim 1. This disclosure also provides a method for purifying a lipopeptide comprising the steps of providing an amorphous preparation of a lipopeptide and crystallizing or precipitating the lipopeptide. In one embodiment, the lipopeptide has a higher degree of purity after crystallization or precipitation than prior to being subjected to crystallization or precipitation. Lipopeptides may be crystallized by hanging drop, sitting drop or sandwich drop vapor diffusion, liquid-liquid or free interface diffusion, microdialysis or dialysis, slow solvent evaporation, sublimation, or microbatch or batch crystallization. In general, a lipopeptide may be precipitated in a similar way, preferably a lipopeptide is precipitated by batch precipitation. The crystallized or precipitated lipopeptide is daptomycin.

Lipopeptides may be crystallized or precipitated following the teachings of this specification. In one embodiment, alipopeptide can be crystallized or precipitated by providing a solution comprising daptomycin with isopropanol, a pH buffering agent and a salt comprising a divalent calcium cation and allowing precipitation or crystallization to occur, as discussed further *infra.* In another embodiment, the salt has buffering capacity such that an additional pH buffering agent does not have to be present in the solution. In a preferred embodiment, the solution provided does not include PEG or PEG-MME or chemically similar compounds. In an embodiment, the method for precipitating or crystallizing the lipopeptide generally comprises the steps of
a) mixing daptomycin in a buffered solution, a salt comprising a calcium divalent cation, and isopropanol at a pH of 5.9 to 6.3; and
b) allowing the lipopeptides to precipitate or crystallize from the solution under the appropriate temperature conditions.

The samples may be monitored, *inter alia,* for crystal or precipitate formation by microscopic examination and the yield may be followed spectrophotometrically. The crystallized or precipitated lipopeptide is daptomycin.

In another embodiment, the lipopeptide can be crystallized by providing a solution comprising isopropanol, a salt comprising a calcium divalent cation and an organic precipitant as discussed further infra. The crystallized lipopeptide is daptomycin. In general, for batch crystallization, the lipopeptide is dissolved in a solution and low molecular weight alcohols, salts comprising a calcium divalent cation, buffers and/or organic precipitants are added to the solution. The samples are then crystallized under the appropriate temperature conditions, with or without stirring. The samples may be monitored, *inter alia,* for crystal formation by microscopic examination and the yield may be followed spectrophotometrically.

As discussed above, daptomycin is crystallized or precipitated in the presence of isopropanol.

Salts include, *inter alia,* magnesium or sodium formate, ammonium sulfate, ammonium dihydrogen phosphate, calcium acetate, zinc acetate, tri-sodium citrate dihydrate, magnesium acetate, sodium acetate, magnesium chloride, cadmium chloride, ammonium acetate, sodium chloride and lithium sulfate. The salt comprises a calcium divalent cation. In one embodiment, the salt is calcium chloride or calcium acetate. In a preferred embodiment, the salt is calcium acetate. Examples of other salts that comprise a divalent cation, such as a calcium cation, are known in the art, and include, *inter alia,* those listed in the 2000 Sigma catalog. Without wishing to be bound to any theory, it is thought that the salt cation may neutralize the negative charges on the lipopeptide, e.g., the four carboxylic acids of daptomycin. Organic precipitants include, *inter alia,* polyethylene glycols (PEGs) that can vary in average molecular weight from between 300 and 10,000, or polyethylene glycol monomethyl ether (PEG-MME). In a preferred embodiment, the organic precipitant is PEG 300, PEG 600, PEG 2000, PEG 4000, PEG 8000 or PEG 10,000.

The buffered solution has a pH of 5.9 to pH 6.3. In one embodiment, the buffered solution may be obtained by using a pH buffering agent Examples of pH buffering agents include, without limitation, Tris, phosphate, citrate, HEPES, CHES, sodium acetate or 2-morpholinoethanesulfonic acid (MES), sodium borate, sodium cacodylate, imidazole and tri-sodium citrate dihydrate. In a more preferred embodiment, the pH buffer is calcium acetate pH 6.1, sodium acetate pH 6.1, MES pH 6.0 and calcium acetate pH 6. In another embodiment, the solution may be buffered by using a salt that also has buffering capacity. In a preferred embodiment, the pH buffer is calcium acetate pH 6.1.

There is disclosed a lipopeptide precipitated or crystallized using hanging drop vapor diffusion from a solution containing 2 to 40% low molecular weight or polyhydric alcohol, 0.001 to 0.5 M salt and 0.005 to 0.2 M pH buffering agent. There is disclosed preferably a lipopeptide precipitated or crystallized from a solution containing 3 to 30% low molecular weight or polyhydric alcohol, 0.01 to 0.3 M salt and 0.01 to 0.1 M pH buttering agent. There is disclosed more preferably a lipopeptide precipitated or crystallized from a solution containing 5 to 20% low molecular weight or polyhydric alcohol, 0.02 to 0.1 M salt and 0.02 to 0.07 M pH buffering agent. The solution provided may or may not include polyethylene glycol (PEG) or polyethylene glycol monomethyl ether (PEG-MME).

There is disclosed a lipopeptide precipitated or crystallized using batch crystallization from a solution containing 65 to 95% low molecular weight or polyhydric alcohol, 0.001 to 0.5 M salt and 0.001 to 0.2 M pH buffering agent. There is disclosed preferably a lipopeptide precipitated or crystallized from a solution containing 70 to 90% low molecular weight or polyhydric alcohol, 0.005 to 0.04 M salt and 0.005 to 0.04 M pH buffering agent. There is disclosed a lipopeptide crystallized from a solution which also comprises 3-8% organic precipitant. There is disclosed more preferably a lipopeptide precipitated or crystallized from a solution containing 80 to 85% low molecular weight or polyhydric alcohol, 0.01 to 0.03 M salt and 0.01 to 0.03 M pH buffering agent. In some embodiments disclosed herein, the solution further comprises about 4 to 5% organic precipitant, e.g., PEG or PEG-MME. In other embodiments disclosed herein, the solution provided does not include polyethylene glycol (PEG) or polyethylene glycol monomethyl ether (PEG-MME).

The lipopeptide is precipitated or crystallized at a temperature of 20-30°C. In a more preferred embodiment, the mixture is crystallized or precipitated at approximately 23-28°C. In an even more preferred embodiment, the mixture is crystallized or precipitated at approximately 27 °C. The mixture may be crystallized or precipitated for any time period that results in crystallization or precipitation, preferably approximately one hour to approximately two weeks. In a preferred embodiment, the mixture is stored for a period of approximately three hours to approximately 24 hours, more preferably approximately 8-18 hours.

Daptomycin crystals or crystal-like particles may have a shape that is, without limitation, needle-like, rod-like, urchin-like, flake-like, plate-like or clusters thereof. In one embodiment, daptomycin crystals or crystal-like particles are urchin-like, rod-like or needle-like. The shape of the crystal or crystal-like particle may be determined, *inter alia,* by optical or electron microscopy. In another embodiment, daptomycin crystals or crystal-like particles may be any size that is at least approximately 0.5 µm in diameter in any one dimension. In a more preferred embodiment, daptomycin crystals or crystal-like particle are at least 5 µm, more preferably at least 10 µm. In an even more preferred embodiment, the daptomycin crystals or crystal-like particles are at least 50 µm, more preferably at least 100 µm. The size of the crystal may be determined by any method known to one having ordinary skill in the art. See, e.g., United States Pharmacopeia (USP), pp. 1965-67.

The properties of a crystalline or crystal-like lipopeptide, namely daptomycin, may be determined by any method known to one having ordinary skill in the art. The properties that can be determined include the crystalline or crystal-like lipopeptide's size, shape, birefringence properties, powder x-ray diffraction properties, solid state NMR properties, melting temperature and stability to heat, light, humidity, and degradation. In a preferred embodiment one having ordinary skill in the art may determine whether a lipopeptide is crystalline by powder x-ray diffraction. Powder x-ray diffraction is highly useful for determining whether a preparation is crystalline when the sample is a randomly-oriented collection of small crystals. Diffraction by a mass of randomly-oriented microcrystals produces a series of fines or rings (dependent of the detector) characteristic of the molecule studied and its structure. In a preferred embodiment, powder diffraction is measured by an Automated Powder Diffraction instrument in order to determine whether a lipopeptide is crystalline. See, e.g., Atkins et al., Physical Chemistry, pp. 710-716 (1978) for a discussion of the Debye-Scherrer method for powder diffraction. Any powder diffractometer instrument known in the art that is equipped with any detector for powder diffraction that known in the art could be used to measure the diffraction pattern.

Although the teachings of the instant specification describe the use of a single crystallization or precipitation step in a process for purifying a lipopeptide, one having ordinary skill in the art following the teachings of the specification may use multiple crystallization or precipitation steps in a process for purifying a lipopeptide. It may be advantageous to employ multiple rounds of crystallization or precipitation as disclosed herein in order to further increase purity of the lipopeptide.

After crystallization or precipitation, one may collect the crystalline material or crystal-like precipitate by any method known in the art. In a preferred embodiment, the crystalline material or crystal-like precipitate is collected by centrifugation or filtration. In an even more preferred embodiment, the crystalline material or crystal-like precipitate is collected by filtration because filtration is easily incorporated into a large-scale process for producing a lipopeptide. After the crystalline material or crystal-like precipitate is collected, it may be washed to remove excess crystallizing or precipitating reagents. Any wash solvent known in the art may be chosen so long as it does not appreciably dissolve the crystalline material or crystal-like precipitate. An example of a wash solvent is provided in Example 12. After the crystalline material or crystal-like precipitate is washed, it may be dried by any method known in the art. Examples of drying methods include air-drying, lyophilization (freeze-drying) or desiccation. In a preferred method, the crystalline material or crystal-like precipitate is desiccated. See, e.g., Example 12. In another embodiment, the crystalline lipopeptide's stability may be determined by its residual antibiotic activity or its degradation The antibiotic activity may be measured in a standard agar-diffusion assay against various bacterial strains. See, e.g., Example 32 of United States Patent 4,537,717. The amount of degradation can be measured by, *inter alia,* HPLC analysis, such as that described in International PCT Publication WO 01/53330, published July 26, 2001. In a preferred embodiment, the stability of the crystalline lipopeptide is greater than that of the amorphous form of the lipopeptide. The stability of the crystalline lipopeptide may be determined by exposing the crystalline lipopeptide and an amorphous form thereof to heat, light, humidity, and measuring the degree of degradation of the crystalline form to that of the amorphous form.

Degradation of the lipopeptide may be measured by determining the biological activity of the lipopeptide or any applicable physical parameter. In one aspect of the disclosure, degradation may be measured by determining a particular biological activity of a lipopeptide after it has been subjected to heat, light, humidity, changes in pH or extreme pH, and comparing it to the same biological activity of the lipopeptide prior to any tests of stability. The amount of degradation may be determined, for example, by determining the percentage of biological activity remaining after the test of stability. The percentage of remaining biological activity may be compared to that of an amorphous form of the lipopeptide that has been subjected to the same test. In one aspect of the disclosure, if the lipopeptide is an antibiotic, the crystalline lipopeptide may be tested for its antibiotic activity both prior to and after a test of its stability and compared to an amorphous form that has been tested prior to and after a degradation test. In a preferred aspect of the disclosure, the lipopeptide is daptomycin or a daptomycin-related lipopeptide, and the biological activity test determines the amount of antibiotic activity of the lipopeptides against gram-positive bacteria.

Degradation of a lipopeptide may also be measured by a physical assay. In one aspect of the disclosure, degradation may be measured by determining the percentage of intact crystalline lipopeptide that remains after a test of its stability. The percentage of remaining intact lipopeptide may be compared to that of an amorphous form of the lipopeptide that has been subjected to the same test for stability. In a preferred aspect of the disclosure, the degradation of the lipopeptide may be measured by HPLC, ultraviolet spectroscopy, infrared spectroscopy, NMR, or mass spectroscopy. In an even more preferred aspect of the disclosure, HPLC is used to determine the percentage of intact lipopeptide that remains after a crystalline form of a lipopeptide has been subjected to a test of its stability.

Without wishing to be bound by any theory, applicants believe that daptomycin is crystallized by the methods described above. However, it is thought that washing and/or drying the daptomycin crystals causes the daptomycin crystalline material to revert to a non-crystalline but still crystal-like form. Nevertheless, even if the methods described above only precipitate rather than crystallize the daptomycin or other lipopeptide, the methods still are advantageous because the methods purify the lipopeptide. Also described is a crystalline or crystal-like lipopeptide produced by the above-described methods. In one embodiment, the crystalline or crystal-like lipopeptide comprises a lower amount of one or more impurities compared to the lipopeptide before crystallization or precipitation. In one embodiment, crystalline or crystal-like lipopeptide is daptomycin that comprises a lower level of anhydro-daptomycin and/or the β-isomer of daptomycin compared to daptomycin before crystallization or precipitation. In another embodiment, crystalline or crystal-like daptomycin comprises a lower level of all impurities compared to amorphous daptomycin. Similarly, in another embodiment, the crystalline or crystal-like lipopeptide is a daptomycin-related lipopeptide, as described above, which comprises a lower level of one or more impurities compared to an amorphous form of the daptomycin-related lipopeptide. In yet another embodiment, the crystalline or crystal-like daptomycin-related - lipopeptide comprises a lower level of all impurities compared to an amorphous form of the daptomycin-related lipopeptide.

The crystalline or crystal-like lipopeptide produced by the method described above likely comprises monovalent or divalent cations and water. In a preferred embodiment, the crystalline or crystal-like lipopeptide is daptomycin or daptomycin-related lipopeptide that comprises a divalent cation. In a more preferred embodiment, the divalent cation is a calcium cation. In an even more preferred embodiment, the crystalline or crystal-like daptomycin or daptomycin-related lipopeptide comprises approximately 1-10% by weight of a divalent calcium cation and approximately 0-15% by weight of water as determined by atomic absorption or thermal gravity analysis. In a further preferred embodiment, the crystalline or crystal-like lipopeptide is daptomycin that comprises approximately 5% by weight of a divalent calcium cation and approximately 10% by weight of water, by HPLC analysis, the purity of the crystalline or crystal-like daptomycin is at least 95%, 96%, 97% or 98% or is any purity between 95-98%, relative to related substances and organic contaminants. Alternatively, the crystalline or crystal-like daptomycin or daptomycin-related lipopeptide comprises a monovalent cation such as sodium. Without wishing to be bound by any theory, it is thought that daptomycin or a daptomycin-related lipopeptide may form a salt with the monovalent or divalent cation when it crystallizes or precipitates.

The crystalline form of the lipopeptide, namely daptomycin, may exhibit an increased solubility in a solution or an increased rate of reconstitution in a solution than an amorphous form of the lipopeptide. One may measure whether the crystalline lipopeptide exhibits an increased solubility or increased reconstitution rate by any method known in the art. For instance, one may dissolve a defined amount of a crystalline lipopeptide in an aqueous solution and measure the concentration of the dissolved lipopeptide and compare it to the concentration of dissolved lipopeptide that has been prepared by dissolving the same amount of amorphous lipopeptide in an aqueous solution. Similarly, one may measure the reconstitution rate of a crystalline lipopeptide by adding the crystalline lipopeptide to an aqueous solution and then measuring the concentration of dissolved lipopeptide over time and comparing it to the reconstitution rate of an amorphous lipopeptide that has been measured in the same way. The concentration of lipopeptide is measured by HPLC.

The methods described above provide for the production of crystalline or crystal-like daptomycin that is more pure than amorphous daptomycin. In one embodiment, the lipopeptide is daptomycin or a daptomycin-related lipopeptide. In another embodiment, daptomycin has a purity of no more than 92% before crystallization and has a purity of at least approximately 95%, 96%, 97% or 98% purity, or any purity between 95-98%, after crystallization or precipitation as a crystal-like lipopeptide. In a still further preferred embodiment, daptomycin has a purity of no more than 90% before crystallization and has a purity of approximately at least 97% or 98% after crystallization.

In another embodiment, the daptomycin has a purity of no more than 80%, preferably no more than 70% and more preferably no more than 60% purity before crystallization or precipitation, and has at least approximately 95%, 96%, 97% or 98% purity, or any purity between 95-98%, after purification. In another embodiment, the daptomycin has a purity of no more than 50%, preferably no more than 40%, more preferably no more than 30% purity before crystallization and has at least approximately 95%, 96%, 97% or 98% purity, or any purity between 95-98%, after purification by crystallization or precipitation. Further preferred is an embodiment in which daptomycin has a purity of no more than 20%, more preferably no more than 15%, even more preferably no more than 10% purity before crystallization and has at least approximately 95%, 96%, 97% or 98% purity, or any purity between 95-98%, after purification.

The lipopeptide is daptomycin. A daptomycin preparation may be obtained by any method disclosed, e.g., in any one United States Patents RE32,333, RE32,455, 4,800,157, RE32,310, RE32,311, 4,537,717, 4,482,487, 4,524,135, 4,874,843, 4,885,243 or 5,912,226. A daptomycin preparation may also be obtained by one of the methods described in International PCT Publication WO 01/53330, published July 26, 2001. After the lipopeptide preparation is prepared, the lipopeptide preparation is crystallized or precipitated following the teachings of the specification described herein to produce a crystalline or crystal-like lipopeptide that is more pure or that contains lower levels of specific impurities, e.g., anhydro-daptomycin, than the lipopeptide preparation from which it is prepared.

### Process for Producing Purified Lipopeptides from Fermentation Cultures

Also described herein is a process combining process chromatography steps and crystallization or precipitation to produce a purified lipopeptide. In a preferred aspect of the disclosure, the method comprises the steps of producing a lipopeptide by any method known in the art, such as fermentation of a naturally-occurring or recombinant organism, and then subjecting the lipopeptide preparation to any one or more purification methods such as microfiltration, anion exchange chromatography, hydrophobic interaction chromatography, and/or size exclusion chromatography (either via traditional size exclusion chromatographic media or via ultrafiltration) to produce a lipopeptide preparation that has been partially purified, and then crystallizing or precipitating the lipopeptide preparation to obtain a purified crystalline or crystal-like lipopeptide. In a preferred embodiment, the lipopeptide is daptomycin or a daptomycin-related lipopeptide. The steps regarding fermentation, microfiltration, anion exchange chromatography, hydrophobic interaction chromatography and ultrafiltration are disclosed in the art, e.g., in any one United States Patents RE32,333, RE32,455, 4,800,157, RE32,310, RE32,311, 4,537,717, 4,482,487, 4,524,135, 4,874,843, 4,885,243 or 5,912,226, in International Publication WO 01/53330, published July 26, 2001.

The method optionally comprises the step of collecting and/or washing the crystalline or crystal-like material after the crystallization or precipitation step. In a preferred embodiment, the crystalline lipopeptide preparation may be collected by filtration. In another embodiment, the crystalline or crystal-like material is dried.

In one embodiment, the purification method comprises fermenting *Streptomyces roseosporus* to obtain a fermentation culture containing daptomycin. In one embodiment, the *S*. *roseosporus* may be fermented as described in United States Patent 4,885,243. In another embodiment, the fermentation conditions in which the A-21978C₀-containing crude product is produced by *Streptomyces roseosporus* is altered in order to increase daptomycin production and decrease impurities and related contaminants produced by the *S*. *raseosporus* fermentation culture as described in International PCT Publication WO 01/53330, published July 26, 2001. The WO 01/53330 publication describes fermenting S. *roseosporus* as described in the '243 patent with the modification that the decanoic acid feed is kept at the lowest levels possible without diminishing the overall yield of the fermentation.

Alternatively, daptomycin may be obtained by fermenting a bacterial strain or other producing organism that recombinantly produces daptomycin. In one embodiment, the recombinant bacterial strain or other recombinant organism comprises the daptomycin biosynthetic gene cluster. In another embodiment, the daptomycin biosynthetic gene cluster or a portion thereof is introduced into the organism or bacterial strain via a bacterial artificial chromosome (BAC). In another embodiment, the recombinant bacterial strain used is *S*. *roseosporus* or *S*. *lividans* comprising a BAC containing the daptomycin biosynthetic gene cluster. United States Provisional Application 60/272,207, filed February 28, 2001 describes the daptomycin biosynthetic gene cluster from *S*. *roseosporus* and uses thereof.

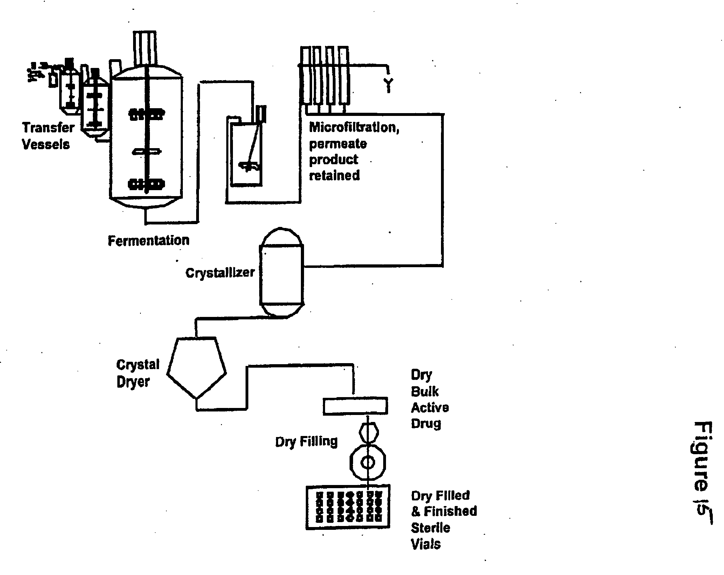

After fermentation, the fermentation broth is clarified by centrifugation, microfiltration or extraction, as is known in the art or as described in the WO 01/53330 publication. In a preferred embodiment, the clarification is performed by microfiltration. See, e.g., Examples 13-16 and Figures 11-15. Figure 11 shows an exemplary manufacturing process that does not use crystallization or precipitation.

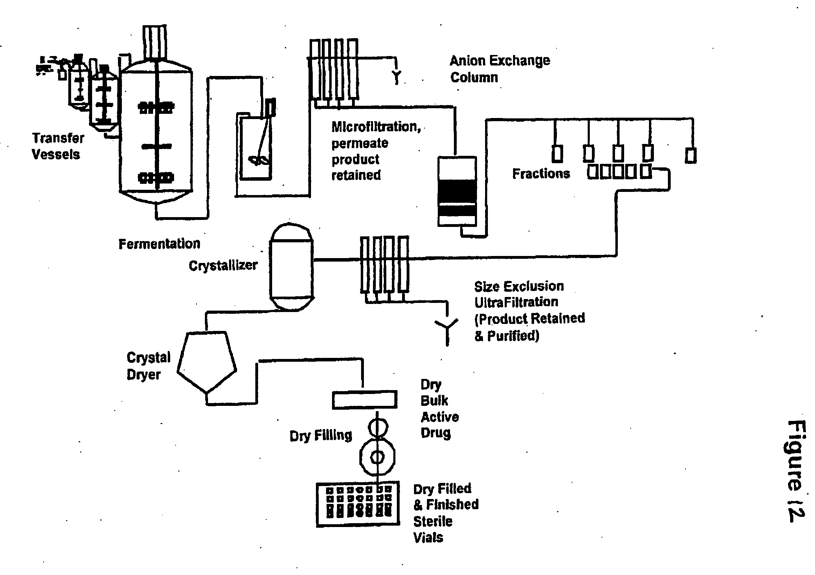

After the fermentation broth is clarified, the concentration of daptomycin in the broth is approximately 5-10%. In one embodiment of the invention, the daptomycin preparation is subjected to a crystallization/precipitation method described above directly subsequent to microfiltration. In one embodiment, crystallization or precipitation is performed under sterile conditions. After crystallization or precipitation is complete, the crystalline or crystal-like daptomycin is optionally collected, washed and dried, as described in further detail below. The dry bulk active drug may then be used to dry fill sterile vials. See, e.g., Example 16 and Figure 12.

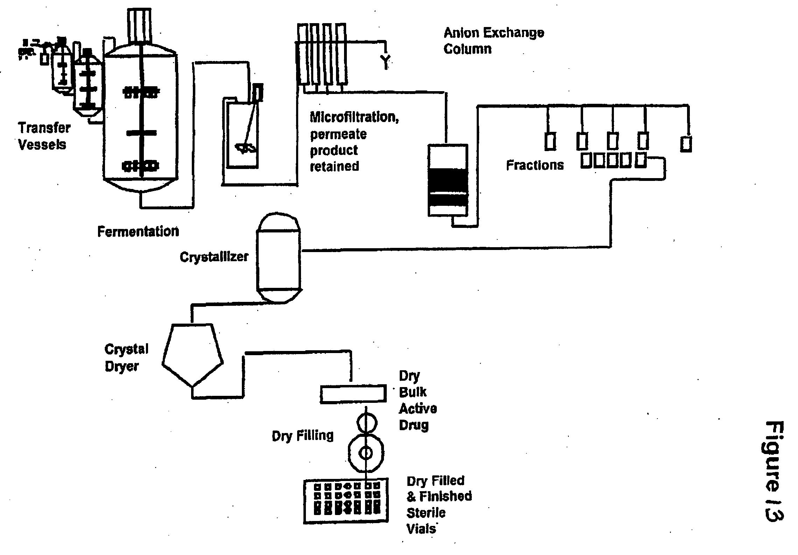

After clarification of the fermentation broth, the lipopeptide may be enriched in the preparation by anion exchange chromatography, as is known in the art or as described in the WO 01/53330 publication or herein. See, e.g., Examples 13-14 and Figures 12-13. After anion exchange chromatography, the purity of daptomycin in the broth is approximately 35-40%. In one embodiment of the invention, the daptomycin preparation is then subjected to a crystallization or precipitation method described above directly subsequent to anion exchange chromatography. In one embodiment, crystallization or precipitation is performed under sterile conditions. After crystallization or precipitation is complete, the crystalline or crystal-like daptomycin is optionally collected, washed and dried as described below. The dry bulk active drug may then be used to dry fill sterile vials. See, e.g., Example 14 and Figure 13.

In another embodiment of the invention, the daptomycin preparation is subjected to size exclusion ultrafiltration after anion exchange chromatography. Size exclusion ultrafiltration is described in the WO 01/53330 Publication The application published July 26, 2001 describes a method of depyrogenating, filtering and concentrating the daptomycin using an ultrafiltration membrane of 10,000 to 30,000 nominal molecular weight (NMW). The application discloses a method in which the lipopeptide passes through the ultrafiltration membrane while large molecular weight impurities, such as endotoxins, are retained by the filter. After the lipopeptide has passed through the membrane, the pH, temperature and/or salt concentration of the lipopeptide solution are altered such that the lipopeptides form micelles. The lipopeptide solution is then filtered on the ultrafiltration membrane under conditions in which the lipopeptide micelles are retained on the membrane while smaller impurities pass through the filter. In this manner, the lipopeptide is further purified. The application discloses the conditions under which lipopeptide micelles may be formed and disassociated as well as methods for filtering the lipopeptide solution to obtain a more purified lipopeptide application. The lipopeptide is daptomycin. The lipopeptide may then be crystallized, as described in the claim. After both anion exchange chromatography and size exclusion ultrafiltration, daptomycin purity is approximately 80-90%. As discussed above, the daptomycin preparation is then subjected to a crystallization/precipitation method described above, preferably under sterile conditions. The crystalline or crystal-like daptomycin may be optionally collected, washed, dried and used to dry fill vials as described below. See, e.g., Example 13 and Figure 12.

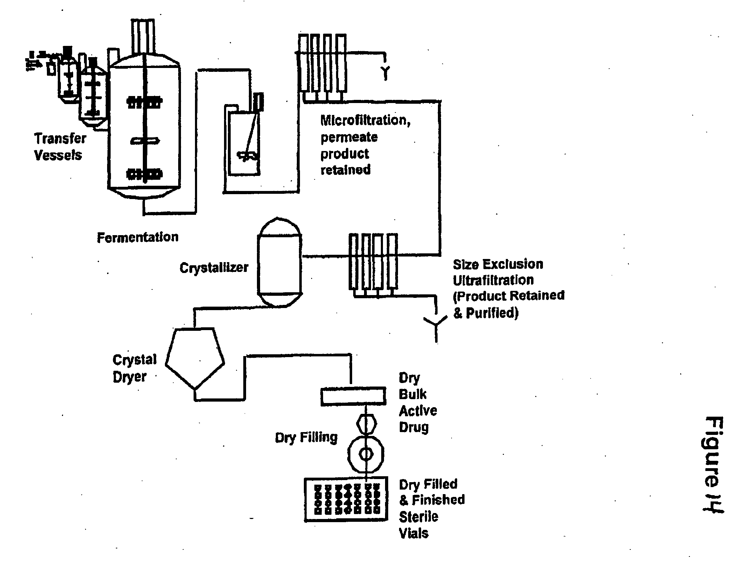

In another embodiment of the invention, the crude daptomycin preparation is subjected to size exclusion ultrafiltration without anion exchange chromatography. After size exclusion ultrafiltration, daptomycin purity is approximately 35-40%. The daptomycin may then be crystallized or precipitated as described in the claims, preferably by sterile methods. As discussed above, the crystalline or crystal-like daptomycin may be collected, washed, dried and used to dry fill sterile vials. See, e.g., Example 15 and Figure 14.

In an alternative embodiment, the lipopeptide preparation is subjected to hydrophobic interaction chromatography (HIC), such as is described in the WO 01/53330 publication, after either the anion exchange chromatography or the size exclusion filtration. The lipopeptide may then be crystallized or precipitated as described in the claims.

After crystallization or precipitation, the crystalline or crystal-like lipopeptide may be collected by a method described herein, e.g., by filtration or centrifugation. The crystalline or crystal-like lipopeptide is optionally washed to remove residual crystallization or precipitation solvent. A method of washing crystals or crystal-like material are described below. See, e.g., Example 3. The washed or unwashed crystal or crystal-like material may be dried. The drying may be performed by any method known in the art, including, without limitation, vacuum drying, spray drying, tray drying or lyophilization. In one embodiment, the drying is performed under sterile conditions. In another embodiment, the drying is performed by vacuum drying. In a more preferred embodiment, the drying is performed using a 0.65 m³ Klein Hastelloy-B double cone vacuum dryer or an equivalent apparatus. The dried crystalline or crystal-like lipopeptide is stable and is easily stored.

In one embodiment, vials are filled with any convenient amount of the dried crystalline or crystal-like daptomycin. In one embodiment, the vials are filled under sterile conditions and then stoppered. In another embodiment, the vials are filled with 50 to 5000 mg each of the dried crystalline or crystal-like daptomycin. In another embodiment, the vials are filled with 100 to 1000 mg each. In another embodiment, the vials are filled with 200 to 500 mg each. In another embodiment, the dried crystalline or crystal-like daptomycin is used for bulk packaging of the daptomycin. The bulk packaging is usually greater than 5000 mg each of the dried crystallize or crystal-like daptomycin. In one embodiment, the bulk packaging is performed under sterile conditions.

In one aspect of the disclosure, the crystallization or precipitation step is performed under sterile conditions. In this embodiment sterile crystallization or precipitation reagents and a sterile, controlled working environment are used. In one embodiment, the lipopeptide is filtered on a ultrafiltration membrane, as disclosed above, before being mixed with the sterile crystallization/precipitation reagents. After crystallization or precipitation, the crystalline or crystal-like lipopeptide preparation is collected by centrifugation or filtration under sterile conditions. In one embodiment, the lipopeptide preparation is collected by sterile filtration. In another embodiment, the crystalline or crystal-like lipopeptide is sterilized after it has been collected. Methods of sterile crystallization, precipitation and filtration as well as methods of sterilizing a final pharmaceutical product are known in the art See, e.g., Remington: The Science and Practice of Pharmacy, Easton, Pennsylvania: Mack Publishing Company (1995), pp. 1474-1487.

In another embodiment, the crystalline or crystal-like lipopeptide, namely daptomycin, is not dried. In this embodiment, the crystalline or crystal-like lipopeptide is preferably stored in a solution that preserves the crystalline or crystal-like nature of the lipopeptide. Vials may be filled with the lipopeptide and solution under sterile or nonsterile conditions. In one embodiment, the conditions are sterile. Alternatively, the crystalline or crystal-like lipopeptide and solution may be used to fill bulk packaging.

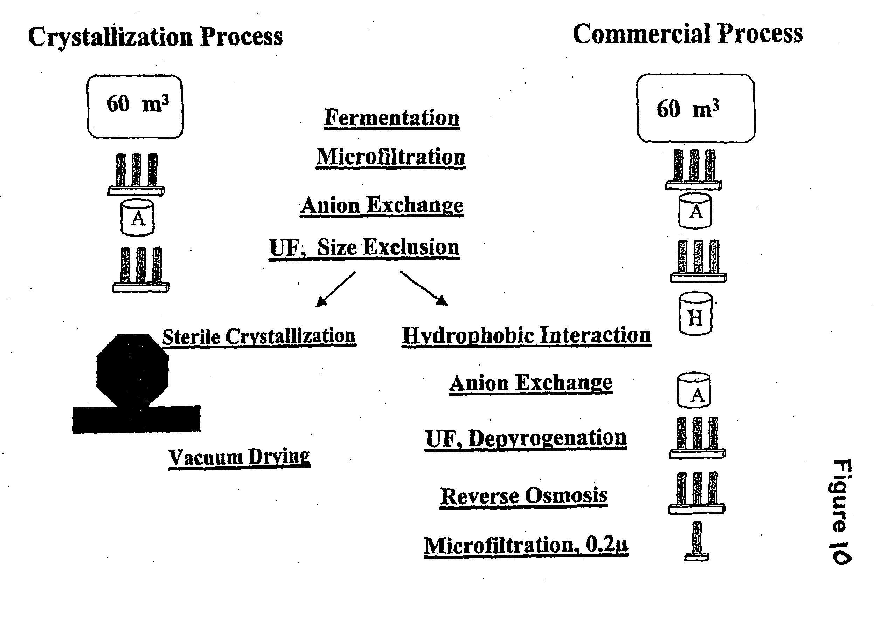

Figures 10 and 11 provide flowcharts describing an exemplary daptomycin manufacturing protocol using crystallization. The incorporation of sterile crystallization into the manufacturing protocol shortens the protocol considerably and eliminates 3 to 4 steps in the process.

### Crystalline or Crystal-like Lipopeptides, Pharmaceutical Compositions and Methods of Use Thereof

Another object of the present disclosure is to provide crystalline or crystal-like lipopeptides or salts thereof, as well as pharmaceutical formulations comprising a crystalline or crystal-like lipopeptide or its salts. The crystalline or crystal-like lipopeptide is daptomycin.

Daptomycin crystals or crystal-like particles, as well as other lipopeptide crystals or crystal-like particles may have a shape such as, *inter alia,* a needle-like shape, a plate-like shape, a lath-like shape, an equant-like shape, an urchin-like shape or a rod-like shape. In one embodiment, daptomycin crystals or crystal-like particles have an urchin-like, needle-like or rod-like shape. The size of the crystals or crystal-like particles may range from approximately 0.5 µm to greater than 100 µm. In one embodiment, the particle size is at least 5 µm or greater. In a more preferred embodiment, the particle size is at least 10 µm or greater, more preferably at least 50 µm. In an even more preferred embodiment, the particle size is at least 100 µm.

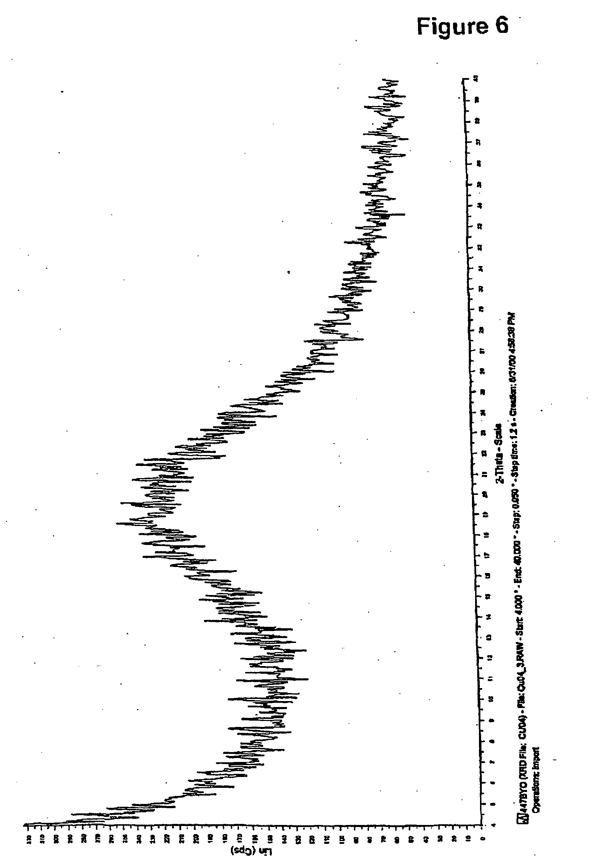

Further, in one embodiment, daptomycin crystals have an x-ray diffraction pattern as shown in Figs. 6, 7 and 8. In another embodiment, the lipopeptide crystal exhibits a different melting point than the amorphous form of the lipopeptide.

In one embodiment, a crystalline form ofa lipopeptide exhibits a stability that is equal to or greater than the amorphous form of the lipopeptide. The crystalline form is daptomycin. In another preferred embodiment, the crystalline lipopeptide is sterile. In another preferred embodiment, the stability of the crystalline lipopeptide is greater than the amorphous form of the lipopeptide. The crystalline lipopeptide may exhibit higher stability to heat, light, degradation or humidity than the amorphous form. The stability of the lipopeptide may be measured by any means including, e.g., antibiotic activity, degradation of the lipopeptide or conversion of daptomycin to anhydro-daptomycin or the β-isomer of daptomycin. In another embodiment, the crystalline form of the lipopeptide may be more quickly reconstituted in aqueous solution than the amorphous form of the lipopeptide.

Crystalline or crystal-like lipopeptides, such as daptomycin or a daptomycin-related lipopeptide, pharmaceutically-acceptable salts, esters, amides, ethers and protected forms thereof, can be formulated for oral, intravenous, intramuscular, subcutaneous, aerosol, topical or parenteral administration for the therapeutic, empirical or prophylactic treatment of diseases, particularly bacterial infections. Reference herein to "crystalline or crystal-like lipopeptides" or "crystalline or crystal-like daptomycin" includes pharmaceutically acceptable salts thereof. Crystalline or crystal-like lipopeptides, such as daptomycin, may be particularly advantageous for pharmaceutical compositions because they can be easily formulated as micronized particles of microspheres, which permits the facile preparation of enterically coated lipopeptides for oral delivery, pharmaceutical compositions for aerosol delivery to, e.g., the lung, and the preparation of lipopeptides formulations for sustained release. Crystalline or crystal-like lipopeptides and crystalline or crystal-like daptomycin may also be more readily dissolved in aqueous solution.

Crystalline or crystal-like lipopeptides, including daptomycin or daptomycin-related lipopeptides can be formulated using any pharmaceutically acceptable carrier or excipient that is compatible with daptomycin or with the lipopeptides of interest. See, e.g., Handbook of Pharmaceutical Additives: An International Guide to More than 6000 Products by Trade Name, Chemical, Function, and Manufacturer, Ashgata Publishing Co., eds., M. Ash and I. Ash, 1996; The Merck Index: An Encyclopedia of Chemicals, Drugs and Biologicals, ed. S. Budavari, annual; Remington's Pharmaceutical Sciences, Mack Publishing Company, Easton, PA; Martindale: The Complete Drug Reference, ed. K. Parfitt, 1999; and Goodman & Gilman's The Pharmaceutical Basis of Therapeutics, Pergamon Press, New York, NY, ed. L S. Goodman et al.; for a general description of the methods for administering various antimicrobial agents for human therapy. Compounds can be mixed with conventional pharmaceutical carriers and excipients and used in the form of tablets, capsules, elixirs, suspensions, syrups, wafers, creams and the like. Compounds may also be mixed with other therapeutic agents and antibiotics, such as discussed herein. The compositions comprising a compound will contain from about 0.1 to about 90% by weight of the active compound, and more generally from about 10 to about 30%.

The compositions can be delivered using controlled (e.g., capsules) or sustained release delivery systems (e.g., bioerodable matrices). Exemplary delayed release delivery systems for drug delivery that are suitable for administration of the compositions are described in U.S. Patent Nos. 4,452,775 (issued to Kent), 5,239,660 (issued to Leonard), 3,854,480 (issued to Zaffaroni).

The compositions may contain common carriers and excipients, such as corn starch or gelatin, lactose, sucrose, microcrystalline cellulose, kaolin, mannitol, dicalcium phosphate, sodium chloride and alginic acid. The compositions may contain croscarmellose sodium, microcrystalline cellulose, corn starch, sodium starch glycolate and alginic acid.

Tablet binders that can be included are acacia, methylcellulose, sodium carboxymethylcellulose, polyvinylpyrrolidone (Povidone), hydroxypropyl methylcellulose, sucrose, starch and ethylcellulose.

Lubricants that can be used include magnesium stearate or other metallic stearates, stearic acid, silicone fluid, talc, waxes, oils and colloidal silica.

Flavoring agents such as peppermint, oil of wintergreen, cherry flavoring or the like can also be used. It may also be desirable to add a coloring agent to make the dosage form more aesthetic in appearance or to help identify the product.

For oral use, solid formulations such as tablets and capsules are particularly useful. Sustained release or enterically coated preparations may also be devised. In another embodiment, crystalline or crystal-like lipopeptides may be supplied in combination with a carrier composition that enhances the oral availability of the lipopeptide. The crystalline or crystal-like lipopeptide is daptomycin. For pediatric and geriatric applications, suspensions, syrups and chewable tablets are especially suitable. For oral administration, the pharmaceutical compositions are in the form of, for example, a tablet, capsule, suspension or liquid. The pharmaceutical composition is preferably made in the form of a dosage unit containing a therapeutically-effective amount of the active ingredient. Examples of such dosage units are tablets and capsules. For therapeutic purposes, the tablets and capsules which can contain, in addition to the active ingredient, conventional carriers such as binding agents, for example, acacia gum, gelatin, polyvinylpyrrolidone, sorbitol, or tragacanth; fillers, for example, calcium phosphate, glycine, lactose, maize-starch, sorbitol, or sucrose; lubricants, for example, magnesium stearate, polyethylene glycol, silica, or talc; disintegrants, for example, potato starch, flavoring or coloring agents, or acceptable wetting agents. Oral liquid preparations generally are in the form of aqueous or oily solutions, suspensions, emulsions, syrups or elixirs may contain conventional additives such as suspending agents, emulsifying agents, non-aqueous agents, preservatives, coloring agents and flavoring agents. Oral liquid preparations may comprise lipopeptide micelles or monomeric forms of the lipopeptide. Examples of additives for liquid preparations include acacia, almond oil, ethyl alcohol, fractionated coconut oil, gelatin, glucose syrup, glycerin, hydrogenated edible fats, lecithin, methyl cellulose, methyl or propyl para-hydroxybenzoate, propylene glycol, sorbitol, or sorbic acid.

For intravenous (IV) use, a water soluble form of a compound can be dissolved in any of the commonly used intravenous fluids and administered by infusion. Intravenous formulations may include carriers, excipients or stabilizers including, without limitation, calcium, human serum albumin, citrate, acetate, calcium chloride, carbonate, and other salts. Intravenous fluids includes without limitation, physiological saline or Ringer's solution. Daptomycin or other lipopeptides also may be placed in injectors, cannulae, catheters and lines.

Formulations for parenteral administration can be in the form of aqueous or non-aqueous isotonic sterile injection solutions or suspensions. These solutions or suspensions can be prepared from sterile powders or granules having one or more of the carriers mentioned for use in the formulations for oral administration. The crystalline or crystal-like lipopeptides can be dissolved in polyethylene glycol, propylene glycol, ethanol, corn oil, benzyl alcohol, sodium chloride, and/or various buffers. For intramuscular, parenteral or intravenous preparations, a sterile formulations of a crystalline or crystal-like lipopeptide compound or a suitable soluble salt form of the compound, for example the hydrochloride salt, can be dissolved and administered in a pharmaceutical diluent such as Water-for-Injection (WFI), physiological saline or 5% glucose. A suitable insoluble form of the crystalline or crystal-like lipopeptide also may be prepared and administered as a suspension in an aqueous base or a pharmaceutically acceptable oil base, e.g., an ester of a long chain fatty acid such as ethyl oleate.

Injectable depot forms may be made by forming microencapsulated matrices of the crystalline or crystal-like lipopeptide in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are also prepared by entrapping the drug in microemulsions that are compatible with body tissues.

For topical use the compounds can also be prepared in suitable forms to be applied to the skin, or mucus membranes of the nose and throat, and can take the form of creams, ointments, liquid sprays or inhalants, lozenges, or troat paints, Such topical formulations further can include chemical compounds such as dimethylsulfoxide (DMSO) to facilitate surface penetration of the active ingredient For topical preparations, a sterile formulation comprising a crystalline or crystal-like lipopeptide, such as crystalline or crystal-like daptomycin, a suitable salt form thereof, may be administered in a cream, ointment, spray or other topical dressing. Topical preparations may also be in the form of bandages that have been impregnated with a lipopeptide composition.

For application to the eyes or ears, the compounds can be presented in liquid or semi-liquid form formulated in hydrophobic or hydrophilic bases as ointments, creams, lotions, paints or powders.

For rectal administration the compounds can be administered in the form of suppositories admixed with conventional carriers such as cocoa butter, wax or other glyceride.

For aerosol preparations, a sterile formulation of a crystalline or crystal-like lipopeptide or a salt form of the compound may be used in inhalers, such as metered dose inhalers, and nebulizers. Aerosolized forms may be especially useful for treating respiratory infections, such as pneumonia and sinus-based infections.

Alternatively, the compounds can be in powder crystalline or ctystal-like form for reconstitution in the appropriate pharmaceutically acceptable carrier at the time of delivery. In another embodiment, the unit dosage form of the compound can be a solution of the compound or a salt thereof in a suitable diluent in sterile, hermetically sealed ampules. The concentration of the compound in the unit dosage may vary, as from about 1 percent to about 50 percent, depending on the compound used and its solubility and the dose desired by the physician. If the compositions contain dosage units, each dosage unit preferably contains approximately from 10-5000 mg of the active material, more preferably 50 to 1000 mg, and even more preferably 100 to 500 mg. For adult human treatment, the dosage employed preferably ranges from 100 mg to 3 g, per day, depending on the route and frequency of administration.

In a further aspect, this disclosure provides a method for treating an infection caused by a gram-positive bacteria in a subject. In a preferred embodiment, the method may be used to treat an infection caused by a gram positive bacteria. The term "treating" is defined as administering, to a subject, a therapeutically effective amount of a compound both to prevent the occurrence of an infection and to control or eliminate an infection, e.g., an established infection. The term "subject", as described herein, is defined as a mammal, a plant or a cell culture. As used herein, the phrase "therapeutically-effective amount" means an amount of daptomycin that prevents the onset, alleviates the symptoms, or stops the progression of a bacterial infection. In a preferred aspect of the disclosure a subject is a human or other animal patient in need of lipopeptide treatment. An established infection may be one that is acute or chronic. An effective dose is generally between about 0.1 and about 75 mg/kg crystalline or crystal-like lipopeptide, such as crystalline or crystal-like daptomycin or daptomycin-related lipopeptide, or a pharmaceutically acceptable salt thereof. A preferred dose is from about 1 to about 25 mg/kg of crystalline or crystal-like daptomycin or a pharmaceutically acceptable salt thereof. A more preferred dose is from about 1 to 12 mg/kg crystalline or crystal-like daptomycin or a pharmaceutically acceptable salt thereof. An even more preferred dose is about 3 to 8 mg/kg crystalline or crystal-like daptomycin or a pharmaceutically acceptable salt thereof. Exemplary procedures for delivering an antibacterial agent are described in U.S. Patent No. 5,041,567, issued to Rogers and in International PCT Publication WO 95/05384.

The crystalline or crystal-like lipopeptide, e.g., daptomycin, can be administered as a single daily dose or in multiple doses per day. The treatment regime may require administration over extended periods of time, e.g., for several days or for from two to four weeks. The amount per administered dose or the total amount administered will depend on such factors as the nature and severity of the infection, the age and general health of the patient, the tolerance of the patient to the lipopeptide and the microorganism or microorganisms involved in the infection. A method of administration is disclosed in WO 00/18419, published April 6, 2000.

The methods comprise administering a compound, or a pharmaceutical composition thereof to a patient in need thereof in an amount that is efficacious in reducing or eliminating the gram-positive bacterial infection. The lipopeptide may be administered orally, parenterally, by inhalation, topically, rectally, nasally, buccally, vaginally, or by an implanted reservoir, external pump or catheter. The lipopeptide may be prepared for opthalmic or aerosolized uses. Compounds, or pharmaceutical compositions thereof also may be directly injected or administered into an abscess, ventricle or joint Parenteral administration includes subcutaneous, intravenous, intramuscular, intra-articular, intrasynovial, cisternal, intrathecal, intrahepatic, intralesional and intracranial injection or infusion. In a preferred embodiment, crystalline or crystal-like daptomycin is administered intravenously, subcutaneously or orally.

The method may be used to treat a patient having a bacterial infection in which the infection is caused or exacerbated by any type of gram-positive bacteria. In a preferred aspect of the disclosure, crystalline or crystal-like daptomycin, daptomycin-related lipopeptide or other lipopeptide, or pharmaceutical compositions thereof, are administered to a patient according to the methods. In another aspect of the disclosure, the bacterial infection may be caused or exacerbated by bacteria including, but not limited to, methicillin-susceptible and methiculin-resistant staphylococci (including *Staphylococcus aureus, Staphylococcus epidermidis, Staphylococcus haemolyticus, Staphylococcus hominis, Staphylococcus saprophyticus,* and coagulase-negative staphylococci), glycopeptide intermediary- susceptible *Staphylococcus aureus* (GISA), penicillin-susceptible and penicillin-resistant streptococci (including *Streptococcus pneumoniae, Streptococcus pyogenes, Streptococcus agalactiae, Streptococcus avium, Streptococcus bovis, Streptococcus lactis, Streptococcus sangius* and *Streptococci* Group C, *Streptococci* Group G and viridans streptococci), enterococci (including vancomycin-susceptible and vancomycin-resistant strains such as *Enterococcus faecalis* and *Enterococcus faecium), Clostridium ditficile, Clostridium clostridiiforme, Clostridium innocuum, Clasfridium perfringens, Clostridium ramosum, Haemophilus influenzae, Listeria monocytogenes, Corynebacterium jeikeium, Bifidobacterium* spp., *Eubacterium aerofaciens, Eubacterium lentum, Lactobacillus acidophilus, Lactobacillus casei, Lactobacilllus plantanum, Lactococcus* spp., *Leuconostoc* spp., *Pediococcus, Peptostreptococcus anaerobius, Peptostreptococcus asaccarolyticus, Peptostreptococcus magnus, Peptostreptococcus micros, Peptostreptococcus prevotti, Peptostreptococcus productus, Propionibacterium acnes,* and *Actinomyces* spp.

The antibacterial activity of daptomycin against classically "resistant" strains is comparable to that against classically "susceptible" strains in *in vitro* experiments. In addition, the minimum inhibitory concentration (MIC) value for daptomycin against susceptible strains is typically 4-fold lower than that of vancomycin. Thus, a compound or a pharmaceutical composition of any one of these crystalline or crystal-like lipopeptides, is administered according to the methods to a patient who exhibits a bacterial infection that is resistant to other antibiotics, including vancomycin. In addition, unlike glycopeptide antibiotics, daptomycin exhibits rapid, concentration-dependent bactericidal activity against gram-positive organisms. Thus, compounds or a pharmaceutical composition of any one of these crystalline or crystal-like lipopeptides, is administered according to the methods to a patient in need of rapidly acting antibiotic therapy.

The method may be used for a gram-positive bacterial infection of any organ or tissue in the body. These organs or tissue include, without limitation, skeletal muscle, skin, bloodstream, kidneys, heart, lung and bone. The method may be used to treat, without limitation, skin and soft tissue infections, bacteremia and urinary tract infections. The method may be used to treat community acquired respiratory infections, including, without limitation, otitis media, sinusitis, chronic bronchitis and pneumonia, including pneumonia caused by drug-resistant *Streptoococcus pneumoniae* or *Haemophilus influenzae.* The method also may be used to treat mixed infections that comprise different types of gram-positive bacteria, including aerobic, caprophilic or anaerobic bacteria. These types of infections include intra-abdominal infections, pneumonia, bone and joint infections and obstetrical/gynecological infections. The method also may be used to treat an infection including, without limitation, endocarditis, nephritis, septic arthritis and osteomyelitis. Any of the above-described diseases may be treated using crystalline or crystal-like daptomycin, daptomycin-related lipopeptide, antibacterial lipopeptide, or pharmaceutical compositions of any one of these crystalline or crystal-like lipopeptides.

Crystalline or crystal-like daptomycin, daptomycin-related lipopeptide or other lipopeptide may also be administered in the diet or feed of a patient or animal. If administered as part of a total dietary intake, the amount of daptomycin or other lipopeptide can be less than 1 % by weight of the diet and preferably no more than 0.5% by weight The diet for animals can be normal foodstuffs to which daptomycin or other lipopeptide can be added or it can be added to a premix.

The method may also be practiced while concurrently administering another form of daptomycin or other lipopeptide antibiotic, e.g., one that is not crystalline or crystal-like, or with one or more antifungal agents and/or one or more antibiotics other than crystalline or crystal-like daptomycin or other crystalline or crystal-like lipopeptide antibiotics. Coadministration of an antifungal agent and an antibiotic other than crystalline or crystal-like daptomycin or another lipopeptide antibiotic may be useful for mixed infections such as those caused by different types of gram-positive bacteria, or those that caused by both bacteria and fungus. Furthermore, crystalline or crystal-like daptomycin or other lipopeptide antibiotic may improve the toxicity profile of one or more co-administered antibiotics. It has been shown that administration of daptomycin and an aminoglycoside may ameliorate renal toxicity caused by the aminoglycoside. In a preferred embodiment, an antibiotic and/or antifungal agent may be administered concurrently with a compound, or in a pharmaceutical composition comprising a compound.

Antibacterial agents and classes thereof that may be co administered with a compound include, without limitation, penicillins and related drugs, carbapenems, cephalosporins and related drugs, aminoglycosides, bacitracin, gramicidin, mupirocin, chloramphenicol, thiamphenicol, fusidate sodium, lincomycin, clindamycin, macrolides, novobiocin, polymyxins, rifamycins, spectinomycin, tetracyclines, vancomycin, teicoplanin, streptogramins, anti-folate agents including sulfonamides, trimethoprim and its combinations and pyrimethamine, synthetic antibacterials including nitrofurans, methenamine mandelate and methenamine hippurate, nitroimidazoles, quinolones, fluoroquinolones, isoniazid, ethambutol, pyrazinamide, paraaminosalicylic acid (PAS), cycloserine, capreomycin, ethionamide, prothionamide, thiacetazone, viomycin, everninomycin, glycopeptide, glycylcylcline, ketolides, oxazolidinone; imipenen, amikacin, netilmicin, fosfomycin, gentamicin, ceftriaxone, Ziracin, LY 333328, CL 331002, HMR 3647, Linezolid, Synercid, Aztreonam, and Metronidazole, Epiroprim, OCA_983, GV_143253, Sanfetrinem sodium, CS_834, Bispenem, A_99058.1, A_165600, A_179796, KA 159, Dynemicin A, DX8739, DU 6681; Ceflupronam, ER 35786, Cefoselis, Sanfetrinem celexetil, HGP_31, Cefpirome, HMR_3647, RU_59863, Mersacidin, KP 736, Rifalazil; Kosan, AM 1732, MEN 10700, Lenapenem, BO 2502A, NE_1530, PR 39, K130, OPC 20000, OPC 2045, Veneprim, PD 138312, PD 140248, CP 111905, Sulopenem, ritipenam acoxyl, RO_65 5788, Cyclothialidine, Sch_40832, SEP_132613, micacocidin A, SB_275833, SR_15402, SUN A0026, TOC 39, carumonam, Cefozopran, Cefetamet pivoxil, and T 3811.

In a preferred embodiment, antibacterial agents that may be co administered with a compound include, without limitation, imipenen, amikacin, netilmicin, fosfomycin, gentamicin, ceftriaxone, teicoplanin, Ziracin, LY 333328, CL 331002, HMR 3647, Linezolid, Synercid, Aztreonam, and Metronidazole.

Antifungal agents that may be co administered with a compound include, without limitation, Caspofungen, Voriconazole, Sertaconazole, IB_367, FK_463, LY_303366, Sch_56592, Sitafloxacin, DB_289 polyenes, such as Amphotericin, Nystatin, Primaricin; azoles, such as Fluconazole, Itraconazole, and Ketoconazole, allylamines, such as Naftifine and Terbinafine; and anti-metabolites such as Flucytosine. Other antifungal agents include without limitation, those disclosed in Fostel et al., Drug Discovery Today 5:25-32 (2000). Fostel et al. disclose antifungal compounds including Corynecandin, Mer_WF3010, Fusacandins, Artrichitin/LL 15G256(, Sordarins, Cispentacin, Azoxybacillin, Aureobasidin and Khafrefungin.

Compounds or a pharmaceutical composition of any one or more of these crystalline or crystal-like lipopeptides, may be administered according to this method until the bacterial infection is eradicated or reduced. In one embodiment, the crystalline or crystal-like lipopeptide is administered for a period of time from approximately 3 days to approximately 6 months. In a preferred embodiment, the crystalline or crystal-like lipopeptide is administered for 7 to 56 days. In a more preferred embodiment, the crystalline or crystal-like lipopeptide is administered for 7 to 28 days. In an even more preferred embodiment, the crystalline or crystal-like lipopeptide is administered for 7 to 14 days. The crystalline or crystal-like lipopeptide may be administered for a longer or shorter time period if it is so desired. In a preferred embodiment, the lipopeptide is daptomycin or daptomycin-related lipopeptide.

In order that this invention may be more fully understood, the following examples are set forth. These examples are for the purpose of illustration only and are not to be construed as limiting the scope of the invention in any way.

### EXAMPLE 1 (Comparative example)

Daptomycin was prepared by conventional techniques. The daptomycin preparation was a pale yellow amorphous powder, with a solubility at 25 °C of greater than 1 g/mL in water and a solubility of 2.8 mg/mL in ethanol. The amorphous daptomycin preparation was hygroscopic and decomposed at 215 °C.

The remaining examples describe crystallizing or precipitating lipopeptides in the presence or absence of an organic precipitant (e.g., PEG).

### EXAMPLE 2 (Comparative example)

In a microbatch crystallization, 25 µL of a daptomycin stock (20 mg/mL in methanol) was sequentially mixed with 15 µL of reagent stock (200 mM calcium acetate, 0.1 M cacodylate (pH 6.5), 18% [w/v] PEG 8000 and 15 µL ethylene glycol) to give a solution that was 27.59% aqueous component, 45% methanol and 27.5% ethylene glycol. Urchin-like crystals were formed at a yield of 50% with a purity of 98% as measured by HPLC.

### EXAMPLE 3 (Comparative example)

A daptomycin stock was prepared by dissolving 440 mg daptomycin in 1 mL of a buffer containing 25 mM sodium acetate (pH 5.0) and 5 mM CaCl₂ Crystallization was done by the vapor diffusion (hanging drop) method, in which 5 µL of the daptomycin stock was added to 5 µL of 0.1 M tri-sodium citrate dihydrate (pH 5.6), and 35% [v/v] tert-butanol in water to form a drop. The drop was suspended over a reservoir solution (0.1 M tri-sodium citrate dihydrate (pH 5.6), and 35% [v/v] tert-butanol in water) in an air-tight environment until crystallization occurred This method yielded urchin like daptomycin crystals. See, e.g., Fig. 2.

### EXAMPLE 4 (Comparative example)

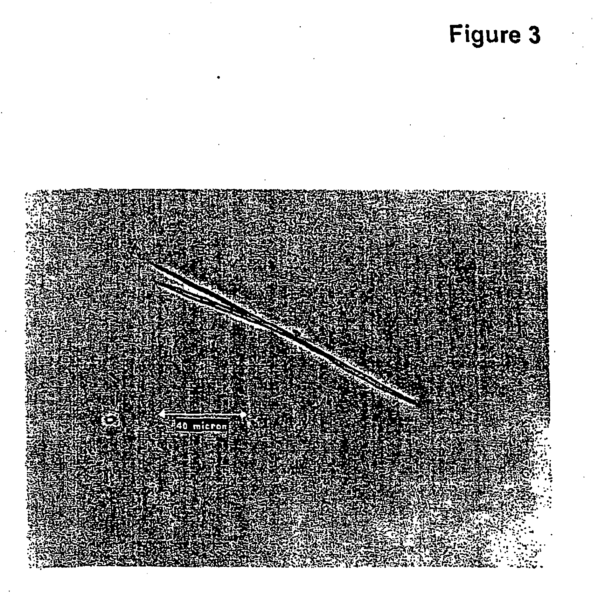

5 µL of a daptomycin stock prepared as in Example 3 was added to 5 µL of a solution containing 0.1 M sodium cacodylate (pH 6.5), 0.2 M calcium acetate and 9% [w/v] PEG 8000 Crystallization was done by the vapor diffusion method as described in Example 3. This method yielded needle-like daptomycin crystals. See, e.g., Fig. 3.

### EXAMPLE 5 (Comparative example)

5 µL of a daptomycin stock prepared as in Example 3 was added to 5 µL of a solution of 0.1 M sodium cacodylate (pH 6.5), 0.2 M zinc acetate and 9% [w/v] PEG 8000 containing 0.1 µL benzamidine to give a final concentration of 220 mg/mL daptomycin. Crystallization was done by the vapor diffusion method as described in Example 3. This method yielded rod-like daptomycin crystals See, e.g., Fig. 4.

### EXAMPLE 6 (Comparative example)

One mL of daptomycin (97.1% pure as determined by HPLC) at a concentration of 20-25 mg/mL in water was sequentially mixed with 231 µL water, 77 µL of calcium acetate (pH 6,0), 960 µL propylene glycol and 231 µL of 50% [w/v] PEG4000, The solution was allowed to sit for 4-5 hours at 4°C. Urchin-like crystals were formed at a yield of 75%. The crystalline daptomycin was washed with isopropanol. The daptomycin was 98.4% pure as determined by BPLC.

### EXAMPLE 7 (Comparative example)

Daptomycin (200 mg 97.1% pure) was dissolved in 2.54 mL water. The daptomycin solution was sequentially mixed in order with 10.0 mL methanol, 0.78 mL 1 M calcium acetate (pH 6.0), 9.50 mL propylene glycol and 2.20 mL 50% [w/v] PEG 4000 to give a final volume of 25.02 mL. The mixture was tumbled at room temperature for 10-14 hours in a hematology mixer (Fischer). Crystals began to appear within a few hours. Final yield was approximately 70-80% after 14 hours. The crystals were harvested by centrifugation at 1000 rpm for 15 minutes. The supernatant was removed and the crystals were resuspended in 12.5 mL isopropanol. The daptomycin suspension was transferred to a column (Biorad) and the isopropanol was removed by allowing it to drip by gravity. The crystals were dried by a nitrogen stream. Any lumps were broken up during the drying procedure to obtain a uniform dry sample. Crystals prepared by this method were urchin-like and had a purity of 98.37%.

### EXAMPLE 8 (Comparative example)

Daptomycin was crystallized according to Example 7 except that PEG 8000 was used in replacement of PEG 4000. The quantities of reagents used are identical to those in Example 7. Crystals prepared by this method were urchin-like and had a purity of 98.84%.

### EXAMPLE 9 (Comparative example)

Two daptomycin samples prepared according to Example 7 and one amorphous sample were analyzed for crystallinity using the USP <695> crystallinity test. Daptomycin particles were mounted in mineral oil on a glass slide and then were examined by polarizing light microscope (PLM). The particles were determined to be crystalline if they were birefringent (have interference colors) and had extinction positions when the stage was rotated.

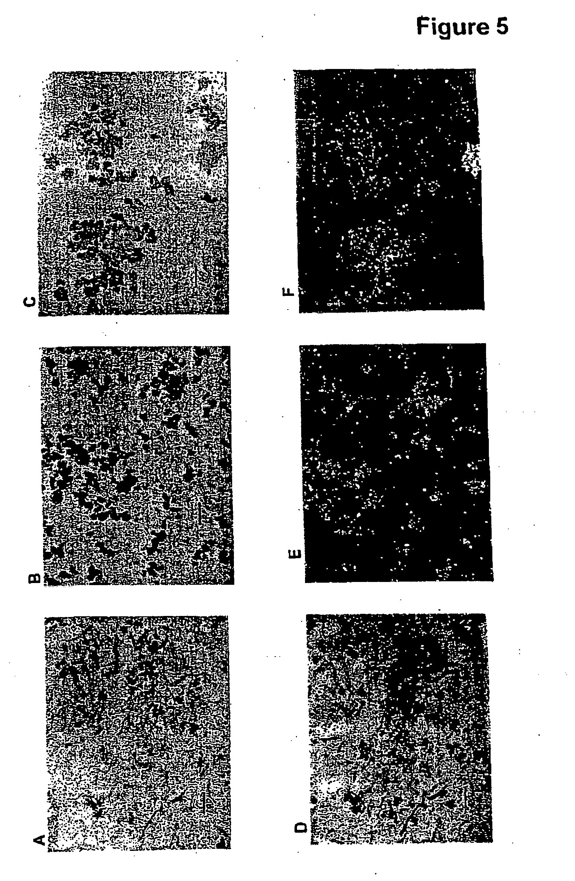

The amorphous daptomycin sample consisted of lacy, flaky particles that were not birefringent. There were a few sliver-like areas in some of the flakes that had weak birefringence, but the particles were primarily amorphous. In contrast, the daptomycin samples prepared according to Example 7 consisted of polycrystalline particles with weak birefringence and some extinction, indicating that they were primarily crystalline. See Fig. 5.

### EXAMPLE 10 (Comparative example)

Two daptomycin samples prepared according to Example 7 and one amorphous sample were analyzed for crystallinity by x-ray powder diffraction The samples were analyzed on a Siemens D500 Automated Powder Diffractometer (ORS 1D No. LD-301-4), which was operated according to ORS Standard Operation Procedure EQ-27 Rev. 9. The diffractometer was equipped with a graphite monochromator and a Cu (λ=1.54 Å) x-ray source operated at 50 kV, 40 mA. Two-theta (θ) calibration is performed using an NBS mica standard (SRM675). The samples were analyzed using the following instrument parameters:

| | |
|---|---|
| Measuring Range for 2θ (degrees) | 4.0 -40.0 |
| Step Width (degrees) | 0.05 |
| Measuring Time per Step (secs) | 1.2 |
| Beam Slits | 1(1°), 2(1°), 3(1°), 4(0.15°), 5(0.15°). |

Sample preparation was performed according to ORS Standard Operation Procedure MIC-7 Rev. 1 using a zero background sample plate.

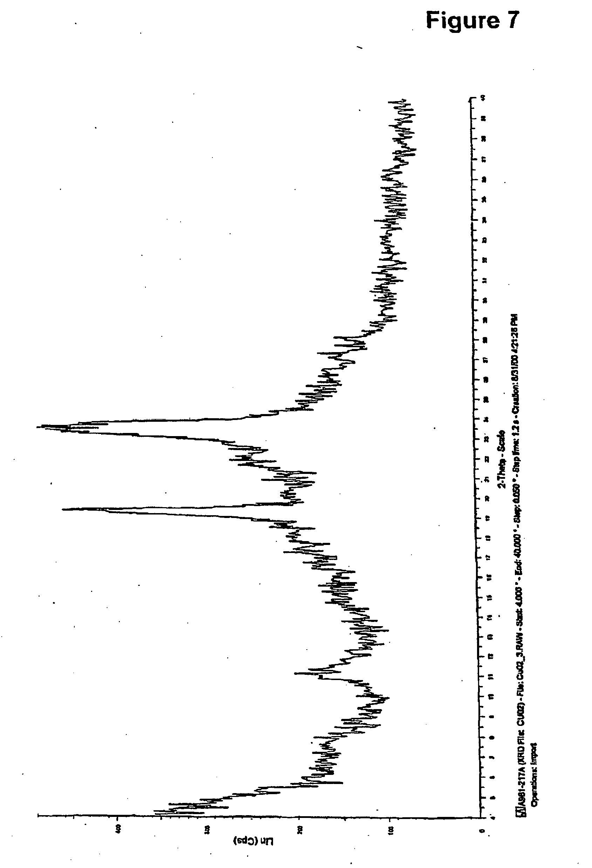

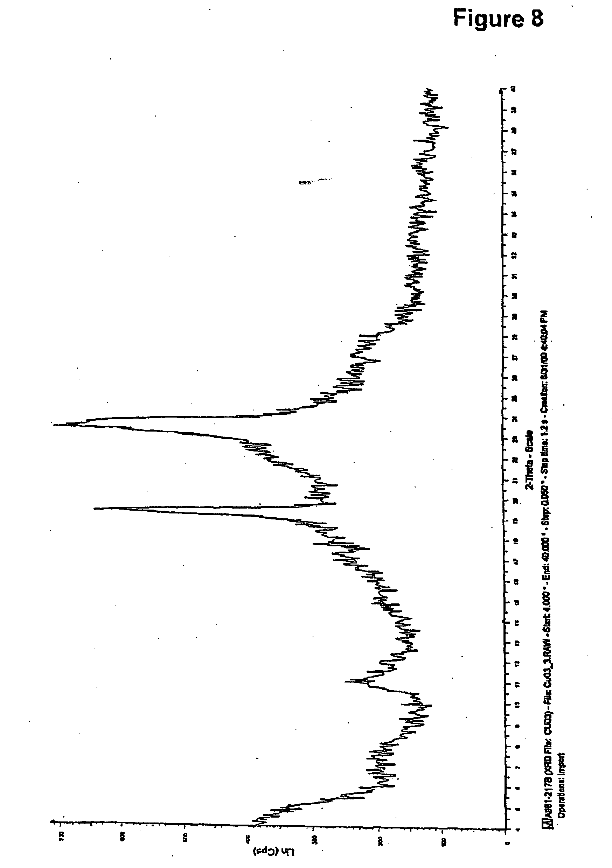

All samples were done using a Cu (λ=1.54 Å) x-ray source. The amorphous daptomycin sample did not show any peaks by x-ray powder diffraction. See Fig. 6. In contrast, the two daptomycin samples both showed peaks by x-ray powder diffraction. The diffraction angle (2θ) of the first daptomycin sample (Fig. 7) was 19.225, 23.242, 23.427 and 23.603 (degree). The diffraction angle (2θ) for the second daptomycin sample (Fig. 8) was 10.966,19.205 and 23.344 (degree). The first crystalline daptomycin sample also showed a small peak between 10-11°. See Fig. 7.

### EXAMPLE 11 (Comparative example)

Daptomycin was dissolved in water. Sodium acetate was added to achieve a final concentration of 187 mM Calcium chloride was added to achieve a final concentration of 28 mM. The daptomycin solution was mixed and isopropanol was added to a final concentration of 78.4%. The solution was mixed and incubated. A precipitated material was formed after incubation. The precipitated material appeared to be urchin-like crystals of approximately 60 µm diameter by optical microscopy. The material was then dried. The dry material contained approximately 30-40% salt. After drying, powder x-ray diffraction was performed. The powder x-ray diffraction did not show the presence of crystals in the dried daptomycin precipitate.

### EXAMPLE 12

One gram of daptomycin (approximately 91.5% purity as measured by HPLC) was added to 16.8 mL of distilled water and dissolved. 2.5 mL of 1M calcium acetate (pH 6.1) and 60 mL of isopropanol was added. The solution was placed in a 27°C water bath and permitted to equilibrate to temperature of the water bath. 5 mL aliquots of isopropanol were slowly added until the solution became cloudy (a total of approximately 30 mL isopropanol). The solution was incubated overnight at 27° C to form a precipitate, The precipitate appeared to contain urchin-like crystals of approximately 60 µm by optical microscopy. See Figure 2.

The daptomycin precipitate was poured into a pressure filter/drying funnel and filtered by gravity. The precipitate was washed twice with 25 mL each time of a washing solution (80% isopropanol and 20% solution A where solution A consists of 18mL of water and 2mL of glacial acetic acid) and allowed to drip by gravity overnight The precipitate was then transferred to a desiccator and dried under vacuum. After drying, powder x-ray diffraction was performed. The powder x-ray diffraction did not show the presence of crystals in the dried daptomycin precipitate. However, purity analysis of the precipitated material by HPLC showed that the material was 98.2% pure daptomycin. Significantly, the daptomycin preparation after precipitation has significantly less anhydro-daptomycin than the daptomycin preparation before precipitation.

Without wishing to be bound by any theory, applicants believe that the conditions used to precipitate the daptomycin in Examples 11 and 12 actually produce a crystalline form of daptomycin but that the subsequent washing steps and/or drying steps cause the crystalline daptomycin to revert to a non-crystalline form. Nonetheless, the non-crystalline daptomycin is still crystal-like as shown in Figure 3 by the birefringence of a crystal sample in polarized light.

### EXAMPLE 13

A fermentation culture of *S*. *roseosporus* NRRL Strain 15998 is conducted in a controlled decanoic acid feed fermentation at levels that optimize the production of the antibiotic while minimizing the production of contaminants. The residual decanoic acid feed is measured by gas chromatography and the target residual level is 10 ppm decanoic acid from the start of induction (approximately at hour 30) until harvest Centrifugation of the culture and subsequent analysis of the clarified broth are used to measure the production of daptomycin by HPLC. The harvest titer is typically between 1.0 and 3.0 grams per liter of fermentation broth

The fermentation culture is harvested either by microfiltration using a Pall-Sep or equivalent microfiltration system, or by full commercial-scale centrifugation and depth filter. The clarified broth is applied to an anion exchange resin, Mitsubishi FP-DA 13, washed with of 30 mM NaCl at pH 6.5 and eluted with of 300 mM NaCl at pH 6.0-6.5. Alternatively, the FP-DA 13 column is washed with of 30 mM NaCl at pH 6.5 and eluted with of 300 mM NaCl at pH 6.0-6.5. The pH is adjusted to 3.0-4.8 and the temperature is adjusted to 2-15 °C. Under these conditions, daptomycin forms a micelle. The micellar daptomycin solution is filtered-washed using a 10,000 NMW ultrafilter (AG Technology Corp. UF hollow fiber or equivalent) in any configuration. The daptomycin micelles are retained by the filter, but a large number of impurities are eliminated because they pass through the 10,000 NMW filter. Ultrafiltration of daptomycin micelles increases daptomycin purity to approximately 80-90%.

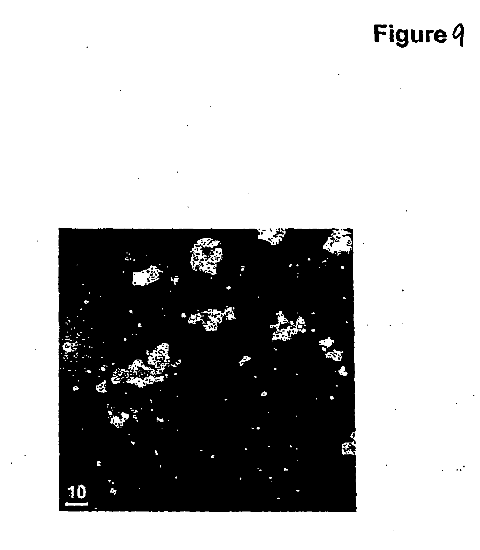

The daptomycin preparation is then crystallized or precipitated under sterile conditions using one of the methods described above. In a preferred embodiment, the daptomycin is crystallized or precipitated according to the protocol described in Examples 7, 8 or 12 except that it can be scaled up for large preparation of daptomycin. The crystalline or crystal-like daptomycin is separated from the crystallization/precipitation solution by filtration, preferably by vacuum filtration. The crystalline or crystal-like daptomycin is washed with washing solution (see Example 3). The crystalline or crystal-like daptomycin is then vacuum dried under sterile conditions using a 0.65 m³ Klein Hastelloy-B double cone vacuum dryer or equivalent apparatus. Vials are then filled with either 250 or 500 mg of dried crystalline daptomycin per vial. Figure 9 shows a flowchart of this manufacturing method.

### EXAMPLE 14

Fermentation of *S*. *roseosporus,* microfiltration of the fermentation culture and anion exchange chromatography is performed as described in Example 13. The daptomycin preparation is approximately 35-40% pure at this point. After anion exchange chromatography, the daptomycin is crystallized or precipitated according to the protocol described in Example 13. The daptomycin is then washed and dried according to the protocol set forth in Example 13. The dried crystalline or crystal-like daptomycin is then used to fill sterile vials as described in Example 13. Figure 6 shows a flowchart of this manufacturing method.

### EXAMPLE 15

Fermentation of *S*. *roseosporus* and microfiltration of the fermentation culture is performed as described in Example 13. After microfiltration, the fermentation culture is subjected to size exclusion ultrafiltration as described in Example 13. The daptomycin preparation is approximately 35-40% pure at this point. After ultrafiltration, the daptomycin is crystallized or precipitated according to the protocol described in Example 13. The daptomycin is then washed and dried according to the protocol set forth in Example 13. The dried crystalline or crystal-like daptomycin is then used to fill sterile vials as described in Example 13. Figure 7 shows a flowchart of this manufacturing method.

### EXAMPLE 16

Fermentation of *S*. *roseosporus* and microfiltration of the fermentation culture is performed as described in Example 13. The daptomycin preparation is 5-10% pure at this point After microfiltration, the fermentation culture is crystallized or precipitated according to the protocol described in Example 13. the daptomycin is then washed and dried and used to fill sterile vials as described in Example 13. Figure 8 shows a flowchart of this manufacturing method.

### EXAMPLE 17 (Comparative example)

CB-131547 (see Figure x), a cyclic lipopeptide analog of daptomycin, was prepared via a semi-synthesis route from daptomycin. The CB-131547 was a pale yellow amorphous powder, with a solubility at 25 °C of ~80 mg/mL in normal saline.

CB-131547 (60 mg, ~90% pure) is dissolved in 2.5 mL water. The CB-131547 solution is sequentially mixed in order with 5.0 mL methanol, 0.2 mL 1 M calcium acetate, (pH 6.0), 2.5 mL propylene glycol, and 1.0 mL 50% (w/v) PEG 4000 to give a final volume of 11.2 mL. The solution is allowed to sit for 4 to 24 hours at 4 °C. CB-131547 crystals are formed at a yield of ~ 70% with a purity ~ 98.0% as determined by HPLC.

### EXAMPLE 18 (Comparative example)

CB-131547 (see Figure x), a cyclic lipopeptide analog of daptomycin, was prepared via a semi-synthesis route from daptomycin. The CB-131547 was a pale yellow amorphous powder, with a solubility at 25 °C of ~80 mg/mL in normal saline.

CB-131547 (60 mg, ~90% pure) is dissolved in 2.5 mL water. 0.2 mL 1 M calcium acetate (pH 6.0) and 8 mL of isopropanol is added. The solution is allowed to equilibrate at room temperature (25 °C) for 5 minutes. One mL aliquots of isopropanol are slowly added until the solution becomes cloudy. The solution is stored at room temperature overnight to form crystals.

## Claims

1. A method of purifying daptomycin, the method comprising
a. mixing daptomycin in a buffered solution comprising a salt comprising a calcium divalent cation and isopropanol at a pH of 5.9 to 6.3 at a temperature of 20-30 degrees C to obtain crystalline or crystal-like daptomycin; and
b. collecting the crystalline or crystal-like daptomycin.

2. The method of claim 1, wherein the solution comprises calcium acetate at a pH of 6.1.

3. The method of any one of claims 1-2, wherein the temperature of the solution is 27 degrees C.

4. The method of any one of claims 1-3, wherein the salt comprising a calcium divalent cation is calcium acetate or calcium chloride.

5. The method of any one of claims 1-4, wherein the lipopeptide is precipitated or crystallized using batch crystallization from a solution comprising 80 to 85% isopropanol, 0.01-0.03 M salt, and 0.01-0.03 M pH buffering agent.

## Patentansprüche

1. Verfahren zum Reinigen von Daptomycin, das Verfahren umfassend:
a. Mischen von Daptomycin in einer gepufferten Lösung, die Salz umfasst, das ein zweiwertiges Calciumkation und Isopropanol mit einem pH-Wert von 5,9 bis 6,3 bei einer Temperatur von 20-30°C umfasst, um ein kristallines oder kristallähnliches Daptomycin zu erhalten; und
b. Auffangen des kristallinen oder kristallähnlichen Daptomycins.

2. Verfahren nach Anspruch 1, wobei die Lösung Calciumacetat mit einem pH-Wert von 6,1 umfasst.

3. Verfahren nach einem der Ansprüche 1-2, wobei die Temperatur der Lösung 27°C beträgt.

4. Verfahren nach einem der Ansprüche 1-3, wobei das ein zweiwertiges Calciumkation umfassende Salz Calciumacetat oder Calciumchlorid ist.

5. Verfahren nach einem der Ansprüche 1-4, wobei das Lipopeptid unter Anwendung von diskontinuierlicher Kristallisation aus einer Lösung, die 80 bis 85% Isopropanol, 0,01-0,03 M Salz und 0,01-0,03 M pH-Puffermittel umfasst, ausgefällt oder kristallisiert wird.

## Revendications

1. Procédé de purification de daptomycine, le procédé comprenant les étapes suivantes:
a. mélanger la daptomycine dans une solution tamponnée comprenant un sel comprenant un cation divalent de calcium et de l'isopropanol à un pH de 5,9 à 6,3 à une température de 20-30 °C pour obtenir de la daptomycine cristalline ou de type cristallin; et
b. recueillir la daptomycine cristalline ou de type cristallin.

2. Procédé selon la revendication 1, dans lequel la solution comprend de l'acétate de calcium à un pH de 6,1.

3. Procédé selon l'une quelconque des revendications 1-2, dans lequel la température de la solution est de 27 °C.

4. Procédé selon l'une quelconque des revendications 1-3, dans lequel le sel comprenant un cation divalent de calcium est de l'acétate de calcium ou du chlorure de calcium.

5. Procédé selon l'une quelconque des revendications 1-4, dans lequel le lipopeptide est précipité ou cristallisé en utilisant une cristallisation en lot à partir d'une solution comprenant de 80 à 85% d'isopropanol, de 0,01 à 0,03 M de sel, et de 0,01 à 0,03 M d'un agent tampon du pH.
